(19) Europäisches Patentamt
European Patent Office
Office européen des brevets

(11) **EP 2 174 948 A1**

(12) **EUROPEAN PATENT APPLICATION**

(43) Date of publication:
**14.04.2010 Bulletin 2010/15**

(51) Int Cl.:
*C07K 7/08* (2006.01)  *C07K 14/16* (2006.01)
*A61P 31/18* (2006.01)

(21) Application number: **08290908.6**

(22) Date of filing: **26.09.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicant: **Centre National de la Recherche
Scientifique**
**75016 Paris Cedex 16 (FR)**

(72) Inventors:
• **Divita, Gilles**
  **34130 Mauguio (FR)**
• **Agopian, Audrey**
  **34090 Montpellier (FR)**
• **Gros, Edwige**
  **34090 Montpellier (FR)**

(74) Representative: **Rançon, Xavier Lucien Abel et al**
**Cabinet Ores**
**36 Rue de Saint Petersbourg**
**75008 Paris (FR)**

(54) **Antiviral polypeptides**

(57) The invention concerns polypeptides derived from the HIV-1 reverse transcriptase which are capable of inhibiting said polymerase and optionally also capable of inhibiting the HIV-1 integrase 3' processing activity, and their therapeutic applications.

**Description**

The invention relates to polypeptides derived from the HIV-1 reverse transcriptase for use as antiviral compounds.

Human immunodeficiency virus type I (HIV-1) is the primary cause of acquired immunodeficiency syndrome (AIDS), a slow progressive and degenerative disease of the human immune system. Despite recent therapeutic developments and the introduction of Highly Active Antiretroviral Therapy (HAART), the rapid emergence of drug-resistant viruses against all approved drugs together with inaccessible latent virus reservoirs and side effects of currently used compounds have limited the efficacy of existing anti-HIV-1 therapeutics (Simon *et al.,* 2006). Therefore, there is still an urgent need for new and safer drugs, active against resistant viral strains or directed towards novel targets in the replicative cycle, which will be useful for multiple drug combination.

HIV-1 reverse transcriptase (RT) plays an essential multifunctional role in the replication of the virus, by catalysing the synthesis of double-stranded DNA from the single-strand retroviral RNA genome (di Marzo Veronese *et al.,* 1986 ; Lightfoote *et al.,* 1986). The majority of the chemotherapeutic agents used in AIDS treatments target the polymerase activity of HIV-1 reverse transcriptase, such as nucleoside reverse transcriptase inhibitors (NRTIs) or non-nucleoside reverse transcriptase inhibitors (NNRTIs) (De Clercq, 2007). The biologically active form of reverse transcriptase is an asymmetric heterodimer that consists of two subunits, p66 and p51 derived from p66 by proteolytic cleavage of the C-terminal RNAse H domain (di Marzo Veronese *et al.,* 1986 ; Lightfoote *et al.,* 1986 ; Restle *et al.,* 1990).

The polymerase domain of both p66 and p51 subunit can be subdivided into four common subdomains: fingers, palm, thumb and connection (Kohlstaedt *et al.,* 1992 ; Jacobo-Molina *et al.,* 1993 ; Huang *et al.,* 1998 ; Rodgers *et al.,* 1995 ; Hsiou *et al.,* 1996). Determination of the three-dimensional structures of reverse transcriptases has revealed that although the folding of individual subdomains is similar in p66 and p51, their spatial arrangement differs markedly (Wang *et al.,* 1994). The p66 subunit contains both polymerase and RNase H active sites. The p66-polymerase domain folds into an "open", extended structure, forming a large active site cleft with the three catalytic residues (Asp$^{110}$, ASp$^{185}$, Asp$^{186}$) within the palm subdomain exposed in the nucleic acid binding site. The primer grip is responsible for the appropriate placement of the primer terminus at the polymerase active site and is involved in translocation of the primer-template (p/t) following nucleotide incorporation (Ghosh *et al.,* 1996 ; Wohrl *et al.,* 1997 and Patel *et al.,* 1995). In contrast, p51 predominantly plays a structural role in the reverse transcriptase heterodimer, by stabilizing the dimer interface thereby favouring loading of the p66 onto the primer-template and maintaining the appropriate enzyme conformation during initiation of reverse transcription (Huang *et al.,* 1992).

In order to propose new classes of HIV inhibitors, extensive efforts have been made in the design of molecules that target protein/protein interfaces required for viral entry, replication and maturation (Divita *et al.,* 1994, 1995a ; Camarasa *et al.,* 2006 and Sticht *et al.,* 2005). The inventors (Divita *et al.,* 1995b and Morris *et al.,* 1999a and 1999b) and others (Restle *et al.,* 1990 and Tachedjian *et al.,* 2001) have proposed that the heterodimeric organization of reverse transcriptase constitutes an interesting target for the design of new inhibitors. The formation of the active heterodimeric HIV-1 reverse transcriptase occurs in a two step process. First a rapid association of the two subunits (dimerization-step) via their connection sub-domains thereby yielding an inactive intermediate-reverse transcriptase, followed by a slow conformational change of this intermediate (maturation-step), generating the biologically active form of this enzyme. The maturation step involves contacts between the thumb of p51 and the RNAse H of p66 as well as between the fingers of p51 with the palm of p66 (Divita *et al.,* 1995b; Cabodevilla *et al.,* 2001; Morris *et al.,* 1999a and Depollier *et al.,* 2005). NNRTIs have been reported to interfere with reverse transcriptase dimerization and to modulate the overall stability of the heterodimeric reverse transcriptase depending on their binding site on reverse transcriptase (Tachedjian *et al.,* 2001; Venezia *et al.,* 2006; Sluis-Cremer, and Tachedjian, 2002; Sluis-Cremer *et al.,* 2002 and Tachedjian and Goff, 2003). NNRTIs including Efavirenz and Nevirapine have been shown to promote HIV-1 reverse transcriptase maturation at the level of the Gag-Pol protein and to affect viral protease (PR) activation, resulting in the suppression of viral release from infected cells (Tachedjian *et al.,* 2005 and Figueiredo *et al.,* 2006). Conversely, NNRTIs such as TSAO and BBNH derivatives act as destabilizers of reverse transcriptase subunit interaction (Sluis-Cremer *et al.,* 2002).

The inventors have demonstrated that preventing or controlling reverse transcriptase dimerization constitutes an alternative strategy to block HIV proliferation and has a major impact on the viral cycle (Morris *et al.,* 1999b). In protein-protein interactions the binding energy is not evenly distributed across the dimer interface but involves specific residues "hot spots" that stabilize protein complexes. The inventors have also shown that the use of small peptides targeting "hot spot" residues required for reverse transcriptase-dimerization constitutes a new strategy to inhibit HIV-1 reverse transcriptase (Divita *et al.,* 1994 and 1995a) and have described a decapeptide "p7" (or "Pep-7") mimicking p66/p51 interface that prevents reverse transcriptase-dimerization by destabilizing reverse transcriptase subunit-interactions and that blocks viral replication (Depollier *et al.,* 2005 and Morris *et al.,* 1999b).

The thumb domain plays an important role in the catalysis and integrity of the dimeric form of reverse transcriptase, thereby constituting a potential target for the design of novel antiviral compounds (Jacobo-Molina *et al.,* 1993; Huang *et al.,* 1998 and Morris *et al.,* 1999a). The p66-thumb domain is involved in primer-template binding and polymerase activity of reverse transcriptase (Jacobo-Molina *et al.,* 1993; Huang *et al.,* 1998 and Wohrl *et al.,* 1997). The p51-thumb domain is required for the conformational changes associated with reverse transcriptase dimer-maturation (Morris *et al.,* 1999a). The Inventors have designed a peptide, Pep-A (SEQ ID NO: 28), derived from a structural motif located between residues 284 and 300, corresponding to the end of helix αI, the loop connecting helices αI and αJ and a part of helix αJ. This peptide is a potent inhibitor of reverse transcriptase interfering with the conformational change associated with full activation of the enzyme. However, although it significantly blocks reverse transcriptase maturation *in vitro,* it lacks antiviral activity (Morris *et al.,* 1999a).

A new generation of HIV inhibitors targeting the third viral enzyme HIV-1 integrase (IN) has been introduced into clinics (Grinsztejn *et al.,* 2007). Integrase is a 32 kDA viral protein that is biologically active under a multimeric form (Cherepanov *et al.,* 2003 and Guiot *et al.,* 2006) . Its oligomeric status is dependent on the reaction catalyzed. A dimeric form is required at each end of the viral DNA end for 3'processing while the tetramer is responsible for the concerted integration (Guiot *et al.,* 2006). Integrase has been reported to be involved in numerous protein/protein interactions with both viral and cellular proteins within the PIC. Integrase plays an essential role in the life cycle of HIV, by catalyzing the insertion of the viral DNA into the host cell chromosome, throughout a multi-steps process taking place during or immediately after reverse transcription of the viral genomic RNA (Craigie, 2001 and Semenova *et al.,* 2008). Integrase binds the viral DNA, thereby forming a nucleoprotein complex, which constitutes the main component of the preintegration complex (PIC). Although the composition of the PIC still remains to be clarified, it contains viral components such as Reverse Transcriptase and Vpr as well as cellular components like BAF, LEDGF-p75, INI1, HMGA1 that stabilize IN/DNA complex, favor its nuclear import and improve integrase mediated viral DNA insertion (Depienne *et al.,* 2000; Farnet *et al.,* 1997; Piller *et al.,* 2003; Popov *et al.,* 1998; Semenova *et al.,* 2008 and Van Maele and Debyser, 2005). Within the PIC, integrase performs 3'-processing in the cytoplasm, which consists in the cleavage of two terminal nucleotides from both 3'-ends of viral DNA. Then, in the nucleus of infected cell, integrase mediates a strand transfer reaction that inserts viral DNA into the cell DNA, resulting in a full-site integration.

The integrity of complexes involving integrase is a requirement for viral replication (Piller *et al.,* 2003 and Semenova *et al.,* 2008). Therefore, altering integrase structural organization and its interactions with partners offer potent targets for the design of new inhibitors. To this aim, extensive efforts have been made to design molecules that target structural organization of the enzyme (Semenova *et al.,* 2008). Inhibitors including monoclonal antibodies, oligonucleotide conjugates and peptides have been proposed to constrain the structure of integrase or to disrupt or prevent oligomeric integrase structure or its complex with DNA (Semenova *et al.,* 2008 and Pommier *et al.,* 2005).

The most promising integrase inhibitors reported so far inhibit the strand transfer reaction, by interacting directly with the active site of the preassembled IN/viral DNA complex (Pommier *et al.,* 2005). Two strand transfer inhibitor compounds, Raltegravir (MK-0518) and Elvitegravir (GS-9137), are currently in clinical trials or have been approved by the FDA (Anker *et al.,* 2008; Grinsztejn *et al.,* 2007; Matsuzaki *et al.,* 2006 and Sato *et al.,* 2006). However, as most of the HIV inhibitors targeting catalytic activity of viral enzyme, strand transfer inhibitors have been reported to interact with human proteins that belong to the same polynucleotidyl transferases family (Dyda *et al.,* 1994), and although they are potent HIV replication inhibitors and highly active on viruses harboring resistance to other antiretroviral classes, these inhibitors rapidly select mutations in integrase gene (Malet *et al.,* 2008 and Shimura *et al.,* 2008). Therefore the development of inhibitors that do not target directly the integrase active site, but its structural organization remains a major challenge.

The Inventors have now designed and evaluated a series of peptides derived from the thumb subdomain of HIV-1 reverse transcriptase using Pep-A as a template. Surprisingly, the Inventors have shown that these peptides exhibit an increased efficiency of the inhibition of reverse transcriptase-polymerase activity of HIV-1 reverse transcriptase compared to Pep-A and, for some of them, further inhibit HIV-1 integrase 3' processing activity. Particularly, the inventors have shown that among these peptides, the peptides named $P_{AW}$, P24 and P27 inhibit reverse transcriptase maturation and abolish viral replication without any toxic side-effects. In addition, the peptides named $P_{AW}$, P16 and P26 also alter the stability and structural organization of the HIV-1 integrase, thus disrupting integrase/partner interactions and preventing its nuclear retention.

Accordingly, in a first aspect, the present invention provides an isolated polypeptide derived from Pep-A peptide (SEQ ID NO: 28) selected amongst

a) peptides consisting of or comprising the amino acid sequence $X_1X_2KWX_3TEX_4X_5PLX_6X_7X_8X_9X_{10}$ (SEQ ID NO: 17)
wherein:

$X_1$ is nothing or G,
$X_2$ is nothing if $X_1$ is nothing, and $X_2$ is T if $X_1$ is G,
$X_3$ is L or A
$X_4$ is W or V,

$X_5$ is I or A if $X_4$ is W, and $X_5$ is W if $X_4$ is V,
$X_6$ is nothing or T,
$X_7$ is nothing if $X_6$ is nothing, and $X_7$ is nothing or A if $X_6$ is T,
$X_8$ is nothing if $X_7$ is nothing, and $X_8$ is E if $X_7$ is A,
$X_9$ is A if $X_6$ is T, and $X_9$ is nothing if $X_6$ is nothing, and
$X_{10}$ is E if $X_6$ is T, and $X_{10}$ is nothing if $X_6$ is nothing,

and

b) peptides consisting of the amino acid sequence SEQ ID NO: 1, or consisting of or comprising an amino acid sequence derived therefrom by the substitution of one of the amino acids at positions 1-3, 5, 6 and 8-14 of SEQ ID NO: 1 by an alanine (A) or a glycine (G), or the substitution of the amino acid at position 4 of SEQ ID NO: 1 by a glycine (G) or a valine (V),

wherein said isolated polypeptide inhibits *in vitro* the HIV-1 Reverse Transcriptase polymerase more efficiently than the peptide Pep-A of amino acid sequence SEQ ID NO: 28.

As used herein, the term "inhibits *in vitro* the HIV-1 Reverse Transcriptase polymerase more efficiently than the peptide Pep-A of amino acid sequence SEQ ID NO: 28" means that the polypeptide on the invention inhibits *in vitro* the HIV-1 Reverse Transcriptase polymerase with an inhibition constant (Ki) inferior to that obtained with the peptide Pep-A. This inhibition can be determined by the method described in Restle *et al.,* 1990 or by any other methods, for example by the method described in Example I (see *infra*).

In a preferred embodiment, said polypeptide consists of or comprises the amino acid sequence $X_1X_2KWX_3TEX_4X_5PLX_6X_7X_8X_9X_{10}$ (SEQ ID NO: 17) as defined above.

In a more preferred embodiment, said amino acid sequence corresponding to SEQ ID NO: 17 is the amino acid sequence $X_1X_2KWLTEX_3X_4PLX_5X_6X_7X_8X_9$ (SEQ ID NO: 34) wherein:

$X_1$ is nothing or G,
$X_2$ is nothing if $X_1$ is nothing, and $X_2$ is T if $X_1$ is G,
$X_3$ is W or V,
$X_4$ is I if $X_3$ is W, and $X_4$ is W if $X_3$ is V,
$X_5$ is nothing or T,
$X_6$ is nothing if $X_5$ is nothing, and $X_6$ is nothing or A if $X_5$ is T,
$X_7$ is nothing if $X_6$ is nothing, and $X_7$ is E if $X_6$ is A,
$X_8$ is A if $X_5$ is T, and $X_8$ is nothing if $X_5$ is nothing, and
$X_9$ is E if $X_5$ is T, and $X_9$ is nothing if $X_5$ is nothing.

In a particular embodiment, said amino acid sequence corresponding to SEQ ID NO: 17 or SEQ ID NO: 34 is selected from the group consisting of the amino acid sequences SEQ ID NO: 18, 19, 23 to 25 and 27.

In another particular embodiment, said amino acid sequence corresponding to SEQ ID NO: 17 is the amino acid sequence SEQ ID NO: 26.

Said polypeptide can further inhibit *in vitro* the HIV-1 integrase 3' processing activity. Non limitative examples of such polypeptides are those consisting of or comprising the amino acid sequences SEQ ID NO: 18, 19 and 24.

As used herein, the term "inhibit *in vitro* the HIV-1 integrase 3' processing activity" means that the polypeptide of the invention abolishes the 3' processing activity of HIV-1 integrase with a concentration between 0.5 μM and 300 μM[1], preferably about 100 μM. The inhibition of the HIV-1 integrase 3' processing activity can be determined by the method described in Guiot *et al.,* 2006 or any other methods, for example by the method described in Example III (see *infra*).

In another embodiment, the amino acid sequence derived from SEQ ID NO: 1 is selected from the group consisting of SEQ ID NO: 2 to SEQ ID NO: 7, and SEQ ID NO: 9 to SEQ ID NO: 15.

Table 1 below shows how the amino acid sequences SEQ ID NO: 1 to SEQ ID NO: 7, SEQ ID NO: 9 to SEQ ID NO: 15, SEQ ID NO: 18 to SEQ ID NO: 19 and SEQ ID NO: 23 to SEQ ID NO: 27 derive from the amino acid sequence SEQ ID NO: 28 (the substitutions are underlined).

| Peptide name | Amino acid sequences | | | | | | | | | | | | | | | | SEQ ID NO: |
|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | |

(continued)

| Peptide name | | | | | | | | | | | | | | | | | | SEQ ID NO: |
|---|---|---|---|---|---|---|---|---|---|---|---|---|---|---|---|---|---|---|
| Pep-A | R | G | T | K | A | L | T | E | V | I | P | L | T | | | A | E | 28 |
| P1 | | G | T | K | A | L | T | E | V | I | P | L | T | E | E | A | E | 1 |
| P2 | | <u>A</u> | T | K | A | L | T | E | V | I | P | L | T | E | E | A | E | 2 |
| P3 | | G | <u>A</u> | K | A | L | T | E | V | I | P | L | T | E | E | A | E | 3 |
| P4 | | G | T | <u>A</u> | A | L | T | E | V | I | P | L | T | E | E | A | E | 4 |
| P5 | | G | T | K | <u>A</u> | L | T | E | V | I | P | L | T | E | E | A | E | 5 |
| P6 | | G | T | K | A | <u>A</u> | T | E | V | I | P | L | T | E | E | A | E | 6 |
| P7 | | G | T | K | A | L | <u>A</u> | E | V | I | P | L | T | E | E | A | E | 7 |
| P9 | | G | T | K | A | L | T | E | <u>A</u> | I | P | L | T | E | E | A | E | 9 |
| P10 | | G | T | K | A | L | T | E | V | <u>A</u> | P | L | T | E | E | A | E | 10 |
| P11 | | G | T | K | A | L | T | E | V | I | <u>A</u> | L | T | E | E | A | E | 11 |
| P12 | | G | T | K | A | L | T | E | V | I | P | <u>A</u> | T | E | E | A | E | 12 |
| P13 | | G | T | K | A | L | T | E | V | I | P | L | <u>A</u> | E | E | A | E | 13 |
| P14 | | G | T | K | A | L | T | E | V | I | P | L | T | <u>A</u> | E | A | E | 14 |
| P15 | | G | T | K | A | L | T | E | V | I | P | L | T | E | <u>A</u> | A | E | 15 |
| | | $X_1$ | $X_2$ | K | W | $X_3$ | T | E | $X_4$ | $X_5$ | P | L | $X_6$ | $X_7$ | $X_8$ | $X_9$ | $X_{10}$ | 17 |
| | | $X_1$ | $X_2$ | K | W | L | T | E | $X_3$ | $X_4$ | P | L | $X_5$ | $X_6$ | $X_7$ | $X_8$ | $X_9$ | 34 |
| $P_{AW}$ | | G | T | K | <u>W</u> | L | T | E | <u>W</u> | I | P | L | T | <u>A</u> | E | A | E | 18 |
| P16 | | G | T | K | <u>W</u> | L | T | E | V | <u>W</u> | P | L | | | | | | 19 |
| P24 | | G | T | K | <u>W</u> | L | T | E | <u>W</u> | I | P | L | | | | | | 23 |
| P26 | | | | K | <u>W</u> | L | T | E | <u>W</u> | I | P | L | T | <u>A</u> | E | A | E | 24 |
| P27 | | G | T | K | <u>W</u> | L | T | E | <u>W</u> | I | P | L | T | | | A | E | 25 |
| P28 | | G | T | K | <u>W</u> | <u>A</u> | T | E | <u>W</u> | <u>A</u> | P | L | T | <u>A</u> | E | A | E | 26 |
| P29 | | | | K | <u>W</u> | L | T | E | <u>W</u> | I | P | L | T | | | A | E | 27 |

Amino acid sequence SEQ ID NO: 1 is derived from Pep-A in that the arginine residue at N-terminus has been deleted.

Amino acid sequences SEQ ID NO: 2-4, 6, 7 and 9-15 correspond to the amino acid sequence SEQ ID NO: 1 in which, respectively, the amino acids residues at positions 1, 2, 3, 5, 6, 8, 9, 10, 11, 12, 13 and 14 have been substituted by an alanine (A) residue.

Amino acid sequence SEQ ID NO: 5 corresponds to the amino acid sequence SEQ ID NO: 1 in which the amino acid residue at position 4 has been substituted by a glycine (G) residue.

Amino acid sequences SEQ ID NO: 17, 18 and 23-27 derive from the amino acid sequence SEQ ID NO: 1, in that at least the amino acid residues at positions 4 and 8 of the amino acid SEQ ID NO: 1 have been substituted by a tryptophan (W) residue.

In another embodiment of the invention, a cysteine (C) residue is added to the N- or C-terminus of the polypeptide, preferably to the C-terminus, to facilitate the covalent coupling of said polypeptide to a compound such as a chromophore (particularly a fluorophore).

In a particular embodiment of the invention, when the polypeptide of the invention consists of the amino acid sequences SEQ ID NO: 1 or an amino acid sequence derived therefrom or SEQ ID NO: 17 or SEQ ID NO: 34, as defined above, then

- said polypeptide can further contain a cysteine residue at the N-terminus or in the case where the polypeptide corresponds to SEQ ID NO: 17 or SEQ ID NO: 34 wherein $X_1$ and $X_2$ are respectively G and T as defined above, then the amino acid residue at position 2 of said polypeptide can be substituted by a cysteine residue, and
- said polypeptide can further contain a cysteine residue at the C-terminus, in order to cyclise at least two polypeptides of the invention together.

Preferred examples of such polypeptides are amino acid sequences SEQ ID NO: 35 to 40.

In another embodiment of the invention, one to three amino acid residues of said polypeptide is in D conformation.

In another embodiment of the invention, the N- or C-terminal amino acid of said polypeptide is in beta-conformation.

Preferably, the substitution of an amino acid by an amino acid in D- or beta conformation as defined above should not result in a change of the secondary structure of said polypeptide.

In another aspect, the present invention provides a polypeptide as defined above coupled to a cell delivery agent.

As used herein, the term "coupled" means that the polypeptide of the present invention (named cargo) and the cell delivery agent are non-covalently or covalently linked together. The polypeptide of the present invention can also be indirectly and covalently linked to said cell delivery agent by a cross-linking reagent either to one of the terminal ends of said polypeptide or to a side chain of one of the amino acids of said polypeptide.

As used herein the term "cell delivery agent" refers to a compound capable of delivering or enhancing the delivery of a polypeptide inside the cells *in vitro* and/or *in vivo* (*i.e.,* facilitating the cellular uptake of a polypeptide). Non-limitative examples of cell delivery agents are cell-penetrating peptides (CPPs), liposomes, nanoparticles and polycationic molecules (such as cationic lipids). Cell delivery agents are well known to one skilled in the art.

As used herein, the term "cell-penetrating peptide" refers to a peptide of less than 40 amino-acids, preferably less than 30 amino acids, derived from natural or unnatural protein or chimeric sequences, and capable to trigger the movement of the polypeptide of the invention (the cargo) across the cell membrane into the cytoplasm. CPPs can be subdivided into two main classes, the first requiring chemical linkage with the cargo, and the second involving formation of stable, non-covalent complexes. CPPs can be either polycationic, essentially containing clusters of polyarginine in its primary sequence or amphipathic.

CPP-based technologies described so far mainly involve the formation of a covalent conjugate between the cargo and the carrier peptide, which is achieved by chemical cross-linking or by cloning followed by expression of a CPP fusion protein (Nagahara *et al,* 1998; Gait, 2003; Moulton and Moulton, 2004; Zatsepin *et al.,* 2005, Joliot and Prochiantz 2004; El-Andaloussi *et al.,* 2005; Murriel and Dowdy, 2006). Non limiting examples of CPPs include peptides derived from Tat (Fawell *et al.,* 1994; Vives *et al.,* 1997; Frankel and Pabo, 1998), Penetratin (Derossi *et al,* 1994), poly-arginine peptide such as the $Arg_8$ sequence (Wender *et al.,* 2000; Futaki *et al.,* 2001), Transportan, (Pooga *et al,* 1998), protein derived peptides such as VP22 protein from Herpes Simplex Virus (Elliott & O'Hare, 1997), pVec (Elmquist *et al.,* 2001), Calcitonin-derived peptides (Schmidt *et al.,* 1998; Krauss *et al.,* 2004), antimicrobial peptides Buforin I and SynB (Park *et al.,* 1998), polyproline sweet arrow peptide (Pujals *et al.,* 2006) as well as peptides combining different transduction motifs (Abes *et al,* 2007) or transduction domains in tandem with protein or oligonucleotide binding domains (Meade and Dowdy, 2008).

Different chemistries are available for stable or cleavable conjugation involving mainly disulfide or thio-esters linkages. According to the stability and efficiency of the cargo, several parameters need to be considered including the type of linkage chemistry, the nature of the spacer (Gait, 2003; Zatsepin *et al.,* 2005; Snyder and Dowdy, 2005). Conjugation methods offer several advantages for *in vivo* applications including rationalization, reproducibility of the procedure, together with the control of the stoechiometry of the CPP-cargo.

In a preferred embodiment, the polypeptide of the present invention is non-covalently coupled to a peptide vector, preferably the peptides Pep-1 (SEQ ID NO: 29) or Pep-3 (SEQ ID NO: 33).

The present invention also provides the use of at least one polypeptide as defined above, preferably a polypeptide comprising or consisting of the amino acid sequences SEQ ID NO: 18 ($P_{AW}$), SEQ ID NO: 23 (P24) or SEQ ID NO: 25 (P27), for inhibiting , *in vitro, ex vivo* or *in vivo* the reverse transcriptase polymerase activity of HIV-1 reverse transcriptase.

The present invention also provides the use of at least one polypeptide selected from the group consisting of the polypeptides comprising or consisting of the amino acid sequences SEQ ID NO: 18, SEQ ID NO: 19 (P16) or SEQ ID NO: 24 (P26), for inhibiting, *in vitro, ex vivo* or *in vivo* the HIV-1 integrase 3' processing activity.

In another aspect, the present invention provides a pharmaceutical composition comprising at least one polypeptide as defined above, preferably selected from the group consisting of the polypeptides comprising or consisting of the amino acid sequences SEQ ID NO: 18, 19, 23, 24 and 25, and at least one pharmaceutically acceptable carrier.

As used herein, the term "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field. Preferred examples of such carriers or diluents include, but are not limited to, water, saline, Ringer's solutions, dextrose solution, and 5% human serum albumin. Peptide vectors, liposomes, cationic lipids and non-aqueous vehicles such as fixed oils may also be used. The use of such media and agents for pharmaceutically active substances is well known in the art. Except insofar as any conventional media or agent is incompatible with a therapeutic agent as defined hereabove, use thereof in the composition of the present invention is contemplated.

In another aspect, the present invention provides a polypeptide as defined above for use as a medicament, preferably as an antiviral agent.

Said polypeptide is useful for treating or preventing a virus infection, preferably a HIV infection, and more preferably a HIV-1 infection.

As used herein, the term "treating" includes the administration of said polypeptide to a patient who has a virus infection, preferably a HIV infection, more preferably a HIV-1 infection, or a symptom of a virus infection, preferably a HIV infection, more preferably a HIV-1 infection, with the purpose to cure, heal, alleviate, relieve, alter, remedy, ameliorate, improve, or affect the infection, the symptoms of said infections.

The term "preventing" means that the progression of a virus infection, preferably a HIV infection, more preferably a HIV-1 infection, is reduced and/or eliminated, or that the onset of the virus infection, preferably the HIV infection, more preferably a HIV-1 infection, is delayed or eliminated, in a subject having been exposed to (*i.e.,* in contact with) said virus.

In another aspect, the present invention provides a method of treating or preventing a virus infection, preferably a HIV infection, more preferably a HIV-1 infection, in a subject, preferably a human, comprising administering an antiviral effective amount of at least one polypeptide or the pharmaceutical composition as defined above to said subject in need thereof.

When at least two polypeptides of the present invention are used for treating or preventing a virus infection as defined above, then they can be administered separately either simultaneously or sequentially (e.g. as a single composition or different compositions).

The polypeptides or the composition of the invention can be used in combination with one or more antiviral drugs known in the art. When a combination use is conducted, the polypeptides or the composition of the invention and the one or more antiviral drugs can be administered separately, simultaneously or sequentially.

In a preferred embodiment of said combination, the polypeptides or the composition of the invention are used synergistically with the peptide Pep-7 (SEQ ID NO: 32).

The present invention also provides an isolated polynucleotide encoding at least one polypeptide as defined above.

The present invention also provides a recombinant expression cassette, comprising said polynucleotide, under the control of a transcriptional promoter allowing the transcription of said polynucleotide in a host cell.

The recombinant expression cassettes of the invention can be inserted in an appropriate vector, such as a virus, allowing the production of a polypeptide of the invention in a host cell.

Thus the present invention also provides a recombinant vector containing said expression cassette.

The present invention also provides a host cell containing a recombinant expression cassette or a recombinant vector as defined above.

The host cell of the present invention can be a prokaryotic cell (*e.g.,* bacteria) or an eukaryotic cell (e.g., yeast).

The present invention is further illustrated by the additional description which follows, which refers to examples illustrating the preparation and properties of polypeptides of the invention.

## Description of the drawings:

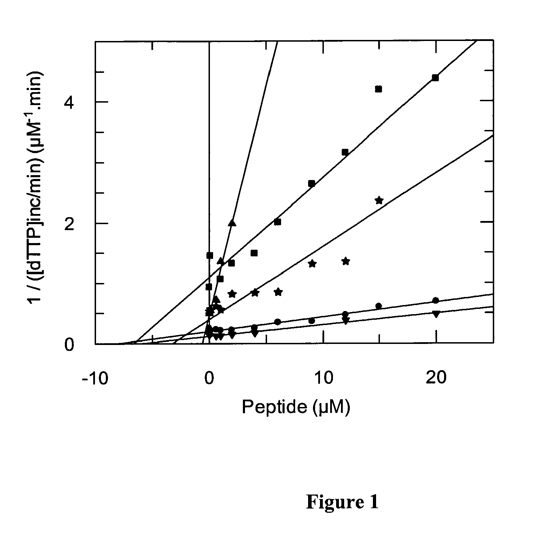

**Figure 1** shows the inhibition of the HIV-1 RT polymerase activity by HIV-1 RT derived peptides. HIV-1 reverse transcriptase (40 nM) was incubated with increasing concentrations of peptides, P1 (SEQ ID NO: 1) (•), P6 (SEQ ID NO: 6) (φ), P10 (SEQ ID NO: 10) (■), P11 (SEQ ID NO: 11) (V) and $P_{AW}$ (SEQ ID NO: 18) (A), then polymerase reaction was initiated by addition of a mix containing $poly(rA).(dT)_{15}$ and dTTP substrates. Inhibition constants were extrapolated from Dixon plots.

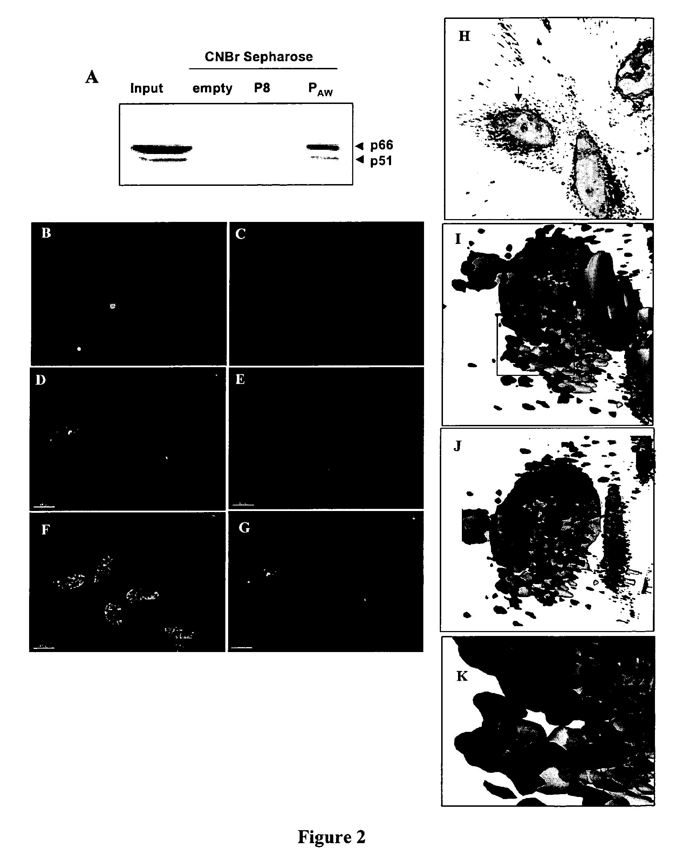

**Figure 2** shows that HIV-1 reverse transcriptase interacts with $P_{AW}$ peptide (SEQ ID NO: 18) in cultured cells. (A) Interaction between $P_{AW}$ and HIV-1 RT monitored by CNBr-pull-down assay. 30 μg (total protein) per lane were separated on 15% SDS-PAGE and subjected to Western blotting using rabbit anti RT antibody. Lanes correspond to control free beads, P8-, $P_{AW}$-beads and total proteins loaded on the gel, respectively. (B-G) Interaction between $P_{AW}$ and HIV-1 RT *in cellulo* was monitored using HeLa cells expressing RT transfected with FITC-$P_{AW}$/Pep-1 complex formed at a 1/10 molar ratio. HIV-1 RT (Alexa 555 secondary antibody) and FITC-$P_{AW}$ are respectively visualized through a Cy3 and a GFP filter. HeLa cells transfected with free FITC-$P_{AW}$ (**B**) or complexed with Pep-1 (**D**). Cultured Hela cells transfected (**E**) or not (**C**) with pcDNA3-p66RT. HeLa cells co-transfected with both pcDNA-p66RT and FITC-$P_{AW}$/Pep-1 at a 1/10 molar ratio. Both RT and $P_{AW}$ display a cytoplasmic localization (**D, E**) and overlay of the RT, FITC-$P_{AW}$, shows co-localization of RT and FITC-$P_{AW}$ (**G**). Nuclear staining with Hoechst dye (**F**). The RT/$P_{AW}$ colocalisation was analyzed by the 3-D image reconstitution with Imaris 6.0 software of 20 frames from z stacking (**G, J**). Global view (**H**), 3D image analysis reveals that RT and $P_{AW}$ co-localize in the cytoplasm at the periphery of the nucleus. 3D analysis of selected cell (arrow) in panel **H**, with (**I**) or without (**J**) nuclear staining. (**K**) Zoom of the box reported in panel **I**.

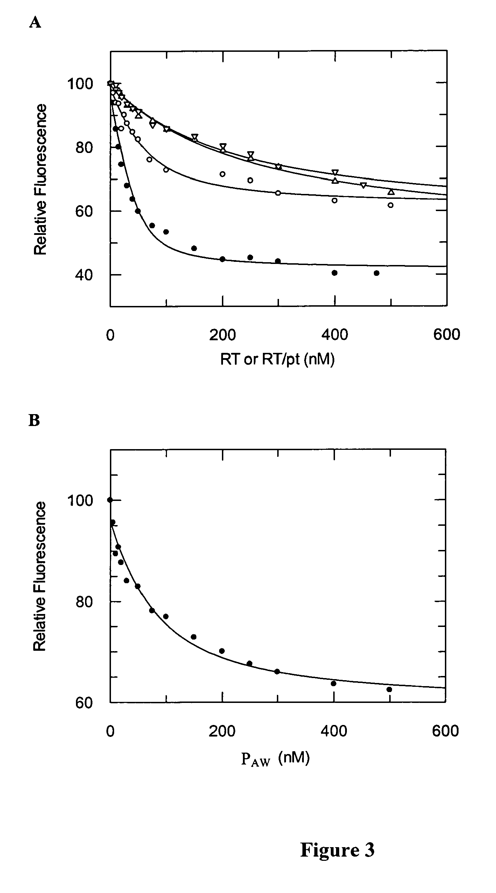

**Figure 3** shows the binding titration of FITC-$P_{AW}$ peptide (SEQ ID NO: 18) to RT and RT-p/t. (**A**) Titration of FITC-$P_{AW}$ binding to RT (○), RT:p/t (•), $p51^{wt}/p_{66}{}^{F61G}$ (△) or $p51^{wt}/p66^{DM}$ (∇). A fixed 200 nM concentration of FITC-$P_{AW}$ was titrated with increasing concentrations of HIV-1 RTs or RT:p/t. The binding of $P_{AW}$ to RT was monitored by following the quenching of extrinsic $P_{AW}$ fluorescence at 512 nm, upon excitation at 492 nm. (**B**) Titration of $P_{AW}$ binding to RT:FAM-p/t. A fixed 20 nM concentration of RT:p/t was titrated with increasing concentrations of $P_{AW}$. The binding of $P_{AW}$ to RT was monitored by following the quenching of extrinsic fluorescence of FAM-labelled p/t at 512 nm, upon excitation at 492 nm. Kd values were calculated using a quadratic equation and correspond to the mean of at least three separate experiments.

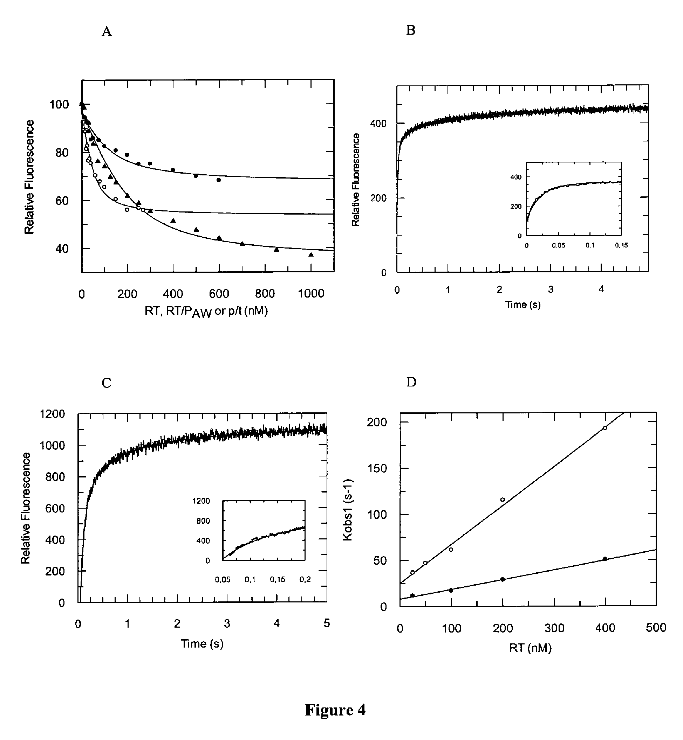

**Figure 4** shows the impact $P_{AW}$ peptide (SEQ ID NO: 18) on the binding of primer/template to HIV-1 RT. (A) Titration of fluorescently labelled p/t binding to RT (○) or RT/$P_{AW}$ (•) and of FITC-$P_{AW}$/RT binding to p/t (A). A fixed 50 nM concentration of fluorescently-labelled p/t was titrated with increasing concentrations of RT or RT/$P_{AW}$. The binding of p/t to RT was monitored by following the quenching of p/t extrinsic fluorescence at 512 nm, upon excitation at 492 nm. A fixed 100 nM concentration of FITC-$P_{AW}$/RT complex was titrated by increasing concentrations of p/t (18/36). The binding of FITC-$P_{AW}$/RT to p/t was monitored by following the quenching of $P_{AW}$ extrinsic fluorescence at 512 nm, upon excitation at 492 nm. Kd values were calculated using a quadratic equation as previously described (Divita *et al.,* 1995b) and correspond to the mean of at least three separate experiments. Kinetics of binding of fluorescently labelled p/t to RT (**B**) and RT/$P_{AW}$ (**C**). Typical stopped-flow time courses are reported, where a fixed 20 nM concentration of FAM-labelled p/t was rapidly mixed with 100 nM of RT (**B**) or RT/$P_{AW}$ (**C**). Data collection acquisition and analysis were performed using KintAsyst 3 software and kinetics were fitted using a three exponential equation. (**D**) Secondary plot of the dependence of the fitted pseudo-first order rate constants for the first phase on RT (○) or RT/$P_{AW}$ (•) concentration.

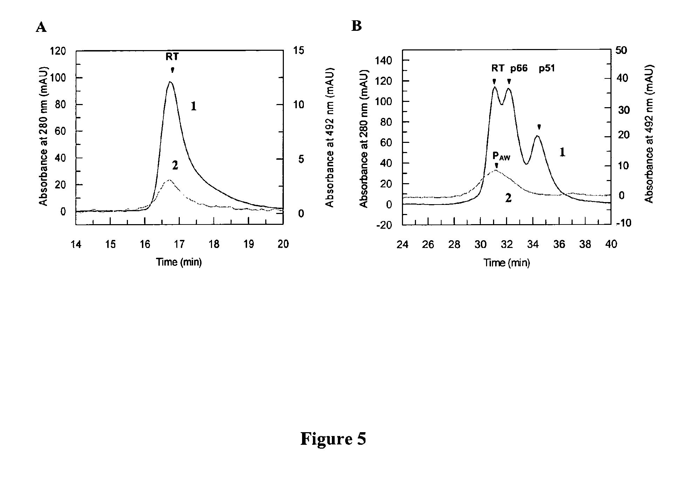

**Figure 5** shows the binding of $P_{AW}$ peptide (SEQ ID NO: 18) to heterodimeric RT as monitored by size-exclusion chromatography. (**A**) Heterodimeric RT (2.3 μM) was incubated in the presence of $P_{AW}$ (10 μM) for 1h30 at room temperature, then applied onto a gel filtration column and eluted with 200 mM potassium phosphate buffer, pH 7.0. (**B**) Heterodimeric RT (10 μM) was incubated in the presence of FITC-$P_{AW}$ (SEQ ID NO: 18) (150 μM) for 2h at room temperature then partially dissociated by 10% acetonitrile for 30 min and analyzed by gel filtration. Proteins were monitored at 280 nm (line 1) and fluorescein-labelled peptide at 492 nm (line 2).

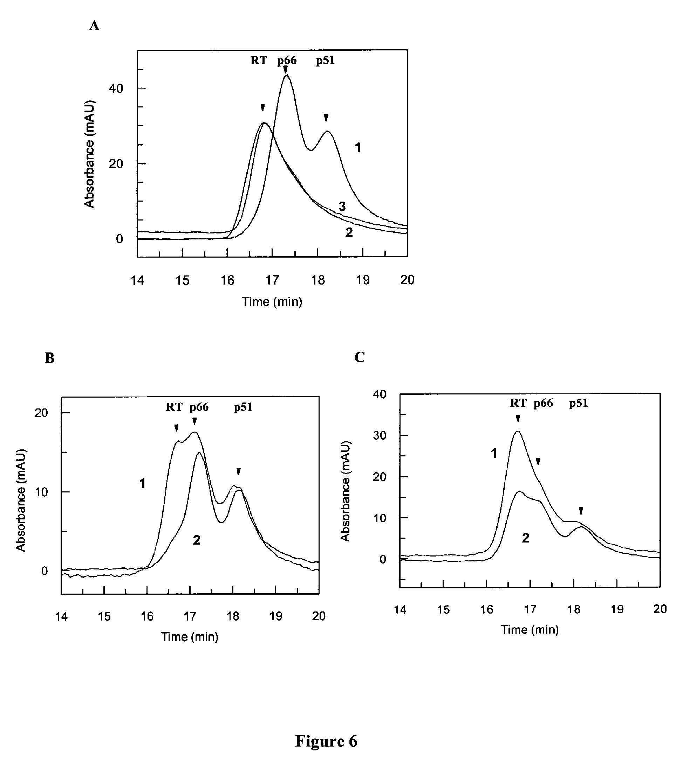

**Figure 6** shows the effect of $P_{AW}$ peptide (SEQ ID NO: 18) on HIV-1 RT-dimerization. (**A**) Impact of $P_{AW}$ on RT-dimerization. 10 μM of RT was dissociated in the presence of 17% acetonitrile yielding 2 peaks corresponding to p66 and p51 subunits (line 1), then p51 and p66 subunits were diluted in an acetonitrile free buffer and incubated overnight at room temperature in the absence (line 2) or presence of 100 μM $P_{AW}$ (line 3). Kinetics of subunit dimerization 30 min (**B**) and 2 hrs (**C**) after dilution in an acetonitrile free buffer. 10 μM of fully dissociated RT were incubated in the presence (line 1) or absence of 100 μM $P_{AW}$ (line 2), then dimerization was induced by dilution in an acetonitrile free buffer and the level of dimer/monomers was monitored at 30 min and 2 hrs by size exclusion chromatography.

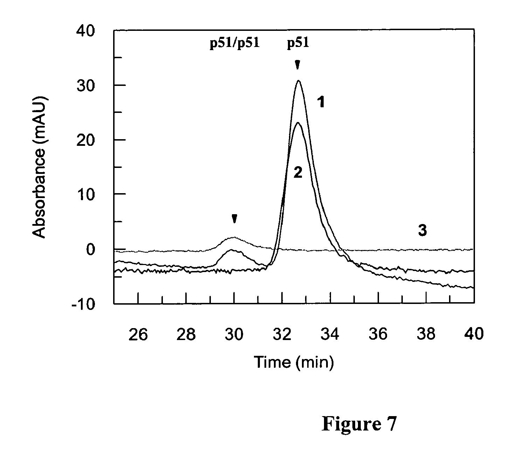

**Figure 7** shows that $P_{AW}$ peptide (SEQ ID NO: 18) favours dimerization of the small p51 subunit. HIV-1 p51 (3.5 μM) free (line 2) or incubated with FITC-$P_{AW}$ (20 μM) (line 1) were applied onto a size exclusion chromatography using two HPLC columns in series. Proteins were monitored at 280 nm and fluorescein-labelled $P_{AW}$ at 492 nm (line 3).

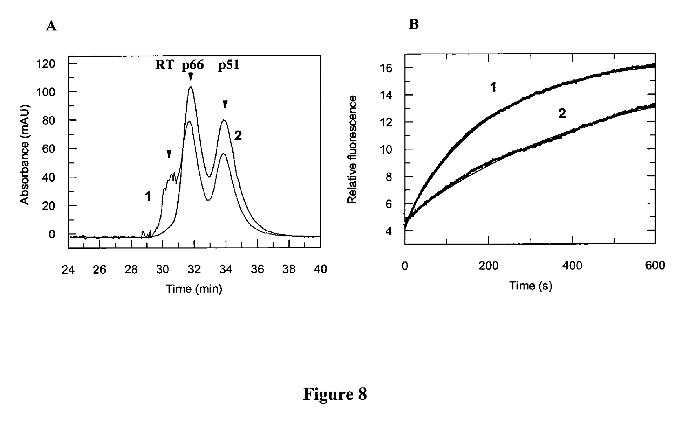

**Figure 8** shows that $P_{AW}$ peptide (SEQ ID NO: 18) prevents HIV-1 RT dissociation. (**A**) $P_{AW}$ associated protection of RT from the acetonitrile dissociation as monitored by size exclusion chromatography. First, HIV-1 RT (8.7 μM) was incubated in the presence (line 1) or absence (line 2) of fluorescently labelled $P_{AW}$ (100 μM) then dissociated by 17% acetonitrile for 30 min at room temperature and applied onto a size exclusion chromatography. (**B**) Kinetics of RT dissociation induced by acetonitrile. RT (0.5 M) was dissociated in the presence of 0.8 μM bis-ANS, by adding 10 % acetonitrile in the absence (blue line) or presence of 5 μM $P_{AW}$ (red line). The kinetics of dissociation were monitored by following the fluorescence resonance energy transfer between tryptophan of RT and Bis-ANS. Tryptophan excitation was performed at 290 nm and the increase of bis-ANS fluorescence emission at 490 nm was detected through a 420 nm cut-off filter. Data acquisition and analysis were performed using KinetAsyst 3 software (Hi-Tech Scientific, Salisbury, England-UK) and traces were fitted according to a single exponential equation.

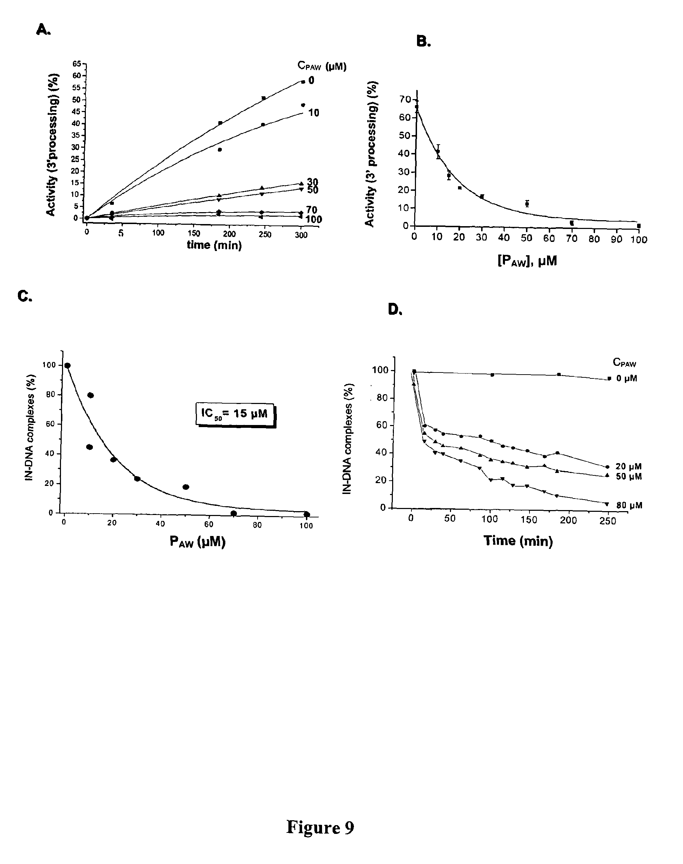

**Figure 9** shows the effect of peptide inhibitors on the DNA-binding step and catalytic activity of HIV-1 Integrase (IN). (**A**) 3'-processing kinetics of IN in the presence of increasing concentrations of $P_{AW}$ (SEQ ID NO: 18). (**B**) Inhibition of 3'- processing activity by $P_{AW}$ (SEQ ID NO: 18). IN activity was allowed to proceed for 300 min before adding SDS. 3'-processing activities as shown in A and B panels were measured by fluorescence anisotropy. (**C**) Quantity of formed integrase-DNA complexes in the presence of increasing concentrations of $P_{AW}$ (SEQ ID NO: 18). All the experiments were performed by pre-incubating integrase and the peptide before addition of the fluorescein-labeled DNA substrate. (**D**) Dissociation of pre-formed integrase-DNA substrate complexes by $P_{AW}$. 4 nM of fluorescein-labeled DNA substrate was preincubated (20 min) with IN (100 nM) in a Tris buffer (20 mM, pH 7.2) containing 50 mM NaCl, 10 mM MgCl2 and 1 mM DTT. $P_{AW}$ was then added (20 μM, 50 μM, 80 μM) and the r value was recorded as a function of time. The relative decrease in the number of complexes was calculated by: (r - rfree ) x 100 / (rt=0 - rfree) wherein r and rt=0 correspond to the measured fluorescence anisotropy values accounting for the number of complexes after and before addition of $P_{AW}$, respectively. rfree corresponds to the fluorescence anisotropy of the free DNA.

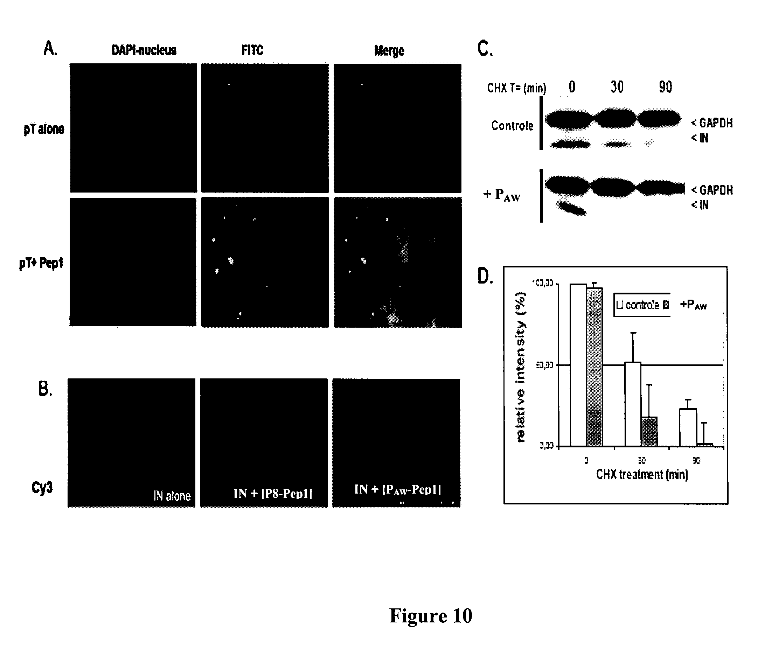

**Figure 10** shows the effect of $P_{AW}$ (SEQ ID NO: 18) on IN localisation and IN stability: (**A**) $P_{AW}$ and P8 (SEQ ID NO: 8) peptides are not able to enter cells alone. When complexed in a non competitive fashion to the cell penetrating peptide Pep-1 (SEQ ID NO: 29), FITC-labelled peptides rapidly localise in the whole cell. (**B**) In Hela cells, stably expressed HA-IN is mostly located in the nucleus. So does it in the presence of P8-Pep-1. It is not the case in the presence of $P_{AW}$-Pep-1. (**C**) HA-IN expressing cells were treated for 0, 30 or 90 min with 100 μg/ml of Cycloheximide, in the presence or not of 1μM $P_{AW}$-Pep-1. A noticeable decrease of IN amount is observed with $P_{AW}$. (**D**) The latter experiment could be further quantified by normalisation with GAPDH expression, suggesting a twice fold decrease in IN half life (from 30 to 18 min).

## EXAMPLE I: DESIGN AND ACTIVITY OF PEPTIDES DERIVED FROM THE THUMB SUBDOMAIN OF HIV-1 REVERSE TRANSCRIPTASE

### 1.1. MATERIALS AND METHODS

#### Materials

Poly(rA)-oligo(dT) and $^3$H-dTTP (1 μCi/μl) were purchased from Amersham Biosciences (Orsay, France). dTTP was from Roche Molecular Biochemicals, Roche Diagnostics (Meylan, France). MF-membrane (25 mm, 0.45 μm) filters for RT assay were purchased from Millipore (Molshein, France).

#### Expression and purification of HIV-1 reverse transcriptase proteins

His-tagged reverse transcriptases (RTs) were expressed and purified as described in Depollier *et al.* (2005) and Agopian *et al.* (2007). M15 bacteria (Qiagen Courtaboeuf, France) were separately transformed with all the constructs of p51 and p66 subunits. Cells were grown at 37 ˚C up to about 0.3 $OD_{595}$, then cultures were cooled to 20˚C and induced overnight with 0.5 mM isopropyl-1-thio-β-D-galactopyranoside. Bacterial cultures expressing His-tagged p66 subunit were mixed with cultures expressing His-tagged p51 subunit to enable dimerization during sonication. For protein isolation and initial purification, the filtered supernatant was applied onto a Hi-Trap chelating column equilibrated with 50 mM sodium phosphate buffer, pH: 7.8, containing 150 mM NaCl supplemented with 50 mM imidazole. The heterodimeric p66/p51 RT was eluted with an imidazole gradient and finally purified by size-exclusion chromatography on a HiLoad 16/60 Superdex 75 column equilibrated with a 50 mM Tris pH: 7.0 buffer containing 1mM EDTA and 50 mM NaCl. Recombinant untagged- HIV-1 $BH_{10}$ RT was expressed in *E. coli* and purified as described in Muller *et al.* (1989). Highly homogeneous preparations from co-expression of the p66 and p51 subunits were stored in -80˚C in buffer supplemented with 50% glycerol. Protein concentrations were determined at 280 nm using a molar extinction coefficient of 260 450 $M^{-1}.cm^{-1}$.

#### Peptide synthesis

Pep-1 (SEQ ID NO: 29) and $P_{AW}$ (SEQ ID NO: 18) were synthesized using an (fluorenylmethoxy)-carbonyl (Fmoc) continuous (Pionner, Applied Biosystems, Foster city, CA) starting from Fmoc-polyamide linker (PAL)-poly(ethyleneglycol) (PEG)-polystyrene (PS) resin at a 0.05 mmol scale. Peptides were purified by semi-preparative reverse-phase high performance liquid chromatography (RP-HPLC; C18 column Interchrom UP5 WOD/25M Uptisphere 300 5 ODB, 250 mm x 21.2 mm) and identified by electrospray mass spectrometry. $P_{AW}$ (1 mM) was coupled to FITC using maleimide-FITC (Molecular Probes. Inc.) (5 mM) through overnight incubation at 4˚C in Phosphate Buffer Saline (PBS: GIBCO BRL). Fluorescently labelled peptide was further purified by RP-HPLC using a C18 reverse-phase HPLC column (Interchrom UP5 HDO/25M Modulo-cart Uptisphere, 250 mm x 10 mm) then identified by electrospray mass spectrometry.

Pep-A (SEQ ID NO: 28), P1 peptide (SEQ ID NO: 1), P1 derived peptides (SEQ ID NO: 2-15 and 19-27) and a scrambled peptide (SEQ ID NO: 16) were purchased from GL Biochem, (Shanghai, China) and Genepep, SA. (Prades le Lez, France).

#### RT-polymerase Assay

RNA-dependent-DNA RT-polymerase activity was measured in a standard reaction assay using poly(rA)-(dT)$_{15}$ as template/primer as described in Restle et *al.* (1990). Ten microliters of reverse transcriptase (RT) at 20 nM was incubated at 37 ˚C for 30 min with 20 μL of reaction buffer (50 mM Tris, pH 8.0, 80 mM KCl, 6 mM $MgCl_2$, 5 mM DTT,

0.15 $\mu$M poly(rA-dT), 15 $\mu$M dTTP, 0.3 $\mu$Ci 3H-dTTP). For peptide evaluation, HIV-1 RT was incubated with increasing concentrations of peptide inhibitors for 23 hrs, and polymerase reaction was initiated by adding reaction buffer. Reactions were stopped by precipitation of nucleic acids with 5 ml of 20% trichloroacetic acid (TCA) solution for 2 h on ice, then filtered using a multiwell-sample collector (Millipore), and washed with 5% TCA solution. Filters were dried at 55˚C for 30 min and radionucleotide incorporation was determined by liquid scintillation spectrometry.

**Antiviral assay**

The anti-HIV activities of the peptides were assayed according to the methods described in Roisin *et al.* (2004). Phytohemagglutinin-P (PHA-P)-activated peripheral blood mononuclear cells (PBMC) treated by increasing concentrations of peptide (from 100 to 0.1 nM), one hour later, were infected with hundred 50% tissue culture infectious doses (TCID50) per 100,000 cells of the HIV-1-LAI strain (Barre-Sinoussi *et al.,* 1983) . This virus was amplified *in vitro* on PHA-P-activated PBMC. Viral stock was titrated using PHA-P-activated PBMC, and 50% $TCID_{50}$ were calculated using Kärber's formula (Kärber, 1931). Samples were maintained throughout the culture, and cell supernatants were collected at day 7 post-infection and stored at -20 ˚C. Viral replication was measured by quantifying reverse transcriptase (RT) activity in cell culture supernatants. In parallel, cytotoxicity of the compounds was evaluated in uninfected PHA-P-activated PBMC by colorimetric 3-(4-5 dimethylthiazol-2-yl)2,5 diphenyl tetrazolium bromite (MTT) assay on day 7 (as described in Mossmann, 1983). Experiments were performed in triplicate and repeated with another blood donor. Data analyses were performed using SoftMax®Pro 4.6 microcomputer software: percent of inhibition of RT activity or of cell viability were plotted vs concentration and fitted with quadratic curves; 50% effective doses ($ED_{50}$) and cytotoxic doses ($CD_{50}$) were calculated.

**I.2. RESULTS**

**Design and evaluation of HIV-1 reverse transcriptase derived peptides**

A series of peptides have been derived from Pep-A sequence (SEQ ID NO: 28), which is derived from HIV-1 reverse transcriptase. First, P1 peptide (SEQ ID NO: 1) was obtained by removing the N-terminal arginine of Pep-A. Then, additional peptides were generated by performing an alanine scan on P1. P1-derived peptides were evaluated using a standard polymerase RT assay. Ki values extrapolated using Dixon plot analysis are reported in Table 2 below. Reported data correspond to the mean of three separate experiments.

Table 2: Inhibition of polymerase activity of HIV-1 RT by Pep-A and P1-derived peptides.

| Peptides | SEQ ID NO: | Amino acid sequence | Ki ($\mu$M) |
|---|---|---|---|
| Pep-A | 28 | RGTKALTEVIPLTEEAE | 35$\pm$ 5 |
| P1 | 1 | GTKALTEVIPLTEEAE | 7.5$\pm$ 2.3 |
| P2 | 2 | ATKALTEVIPLTEEAE | 28$\pm$ 11 |
| P3 | 3 | GAKALTEVIPLTEEAE | 10.3$\pm$ 2.1 |
| P4 | 4 | GTAALTEVIPLTEEAE | 15$\pm$ 2.9 |
| P5 | 5 | GTKGLTEVIPLTEEAE | 20$\pm$ 3.7 |
| P6 | 6 | GTKAATEVIPLTEEAE | 5.7$\pm$ 2.3 |
| P7 | 7 | GTKALAEVIPLTEEAE | 13.5$\pm$ 2.1 |
| P8 | 8 | GTKALTAVIPLTEEAE | 57$\pm$ 19 |
| P9 | 9 | GTKALTEAIPLTEEAE | 15$\pm$ 7.3 |
| P10 | 10 | GTKALTEVAPLTEEAE | 7.3$\pm$ 2.9 |
| P11 | 11 | GTKALTEVIALTEEAE | 7$\pm$ 1.4 |
| P12 | 12 | GTKALTEVIPATEEAE | 22$\pm$ 3 |
| P13 | 13 | GTKALTEVIPLAEEAE | 10.2$\pm$ 2.5 |
| P14 | 14 | GTKALTEVIPLTAEAE | 14$\pm$ 3 |
| P15 | 15 | GTKALTEVIPLTEAAE | 14$\pm$ 2.2 |
| P$_{scramble}$ | 16 | GAKTETLVIPETELEA | 61 $\pm$ 12 |
| P$_{AW}$ | 18 | GTKWLTEWIPLTAEAE | 0.7$\pm$ 0.2 |
| P$_{AW}$-*FITC* | | GTKWLTEWIPLTAEAEC-FITC | 2.7 $\pm$ 0.7 |

All peptides affected the polymerase activity of reverse transcriptase (RT) in a dose-dependent manner, and four peptides P1 (Ki : 7.5 $\mu$M), P6 (Ki : 5.7 $\mu$M), P10 (Ki : 7.3 $\mu$M) and P11 (Ki : 7.0 $\mu$M) possess an inhibition constant lower than 10 $\mu$M (Figure 1).

Pep-A inhibits RT-polymerase activity with an inhibition constant value of 35 $\mu$M. Peptide analysis reveals that, surprisingly, removing the Arg[1] residue in Pep-A increases the potency of the peptide (P1) 5-fold. In comparison to P1, mutation of residues Gly[1], Ala[4], Glu[7], and Leo[11] into alanine significantly affects the potency of the peptide suggesting that the side chains of these residues are required for the interaction with RT. The nature of the side chain of Glu[7] seems to be a major requirement for the interaction with RT as its substitution by alanine (P8), reduces the efficiency of the peptide 8 fold. Lys[3], Thr[6], Val[8] and Glu[14] residues have a minor impact as their mutation into alanine only reduces their potency by a factor of 2. Interestingly, the hydrophobic character of Ala[4] and Val[8] side chains plays a role in the binding of the peptide to RT and reducing their length affects the potency of the corresponding peptides to inhibit RT 2.7- and 2-fold, respectively.

The two residues Ala[4] and Val[8] from P1 were mutated into Trp (W). As shown in Figure 1, the corresponding peptide $P_{AW}$ (SEQ ID NO: 18) significantly inhibits RT polymerase activity with an inhibition constant (Ki) of 0.7 $\mu$M, revealing that mutation of these two residues into Trp improves peptide efficiency 50-fold over Pep-A and 10-fold in comparison to the best lead peptide from the Ala-scan (P6) (see Figure 1, Table 2).

As the interaction between $P_{AW}$ and RT seems to involve both the N-terminal part and Trp-residues of the peptide, the $P_{AW}$ peptidic-sequence was shortened at the N and/or C-terminal extremities and the positional effect of the Trp was evaluated. All peptides were tested in standard RT assays. Results are represented in Table 3 below.

Table 3: Inhibition of polymerase activity of HIV-1 RT by $P_{AW}$-derived peptide sequences.

| Peptides | SEQ ID NO: | Amino acid sequence | Ki ($\mu$M) |
|---|---|---|---|
| $P_{AW}$ | 18 | GTKWLTEWIPLTAEAE | 0.7$\pm$ 0.2 |
| P16 | 19 | GTKWLTEVWPL | 14$\pm$ 4 |
| P17 | 20 | GTKAWTEVWPL | 35$\pm$ 11 |
| P18 | 21 | GTKALTEVIPLT | 53$\pm$ 12 |
| P19 | 22 | GTKAATEVIPLT | 49$\pm$ 9 |
| P24 | 23 | GTKWLTEWIPL | 0.7$\pm$ 0.05 |
| P26 | 24 | KWLTEWIPLTAEAE | 1.8$\pm$ 0.7 |
| P27 | 25 | GTKWLTEWIPLTAE | 0.05$\pm$ 0.01 |
| P28 | 26 | GTKWATEWAPLTAEAE | 2$\pm$ 0.6 |
| P29 | 27 | KWLTEWIPLTAE | 1$\pm$ 0.4 |

Reducing $P_{AW}$ sequence by 2 residues at the N-terminus reduced efficiency 2.5 fold (P26 : Ki= 1.8 $\pm$ 0.7 $\mu$M). In contrast, the 5 last residues at the C-terminus of $P_{AW}$ can be removed without affecting its potency to inhibit RT polymerase activity (P24 : Ki= 0.7 $\pm$ 0.05 $\mu$M). That P18 does not inhibit RT polymerase activity, confirms that the Trp residues form the major interface with RT. Moving Trp[8] to position 9 (P16) and both Trp[4] and Trp[8] to position 5 and 9 (P17) reduced the efficiency of the corresponding peptides 20-fold (Ki : 14 $\pm$ 4 $\mu$M) and 50-fold (Ki : 35 $\pm$ 11 $\mu$M), respectively. Interestingly, removing the last three residues of $P_{AW}$ increases its efficiency 14-fold (P27 : Ki= 50 $\pm$ 0.01 nM).

Results were identical when the peptides of SEQ ID NO: 1-16, 18-27 and 28 further contained a cysteine residue at the C-terminus.

### Antiviral potency of Pep-A derived peptides

Antiviral activity of the 14 peptides P1, P6, P10, P11, $P_{AW}$, P16, P 17, P18, P19, P24, P26, P27, P28 and P29 (respectively SEQ ID NO: 1, 6, 10, 11, 18-27), was evaluated on PHA-P-activated PBMC infected with HIV-1 LAI. Results (shown in Table 4 below) were reported as 50% efficient concentration ($EC_{50}$) and selectivity index (SI) corresponding to the ratio between $EC_{50}$ and the cytotoxic concentration ($CC_{50}$) inducing 50% death of uninfected PBMCs and relative to Pep-A and P8. In order to avoid any limitation due to the poor ability of peptides to cross cellular membranes, they were associated to the peptide-based nanoparticle delivery system Pep-1 (SEQ ID NO: 29), at a 1/10 molar ratio. Pep-1 has been successfully used for the delivery of peptides and proteins, into numerous cell lines as well as *in vivo* (Gros *et al.,* 2006 and Morris *et al.,* 2001). The inability of free peptides to block viral replication is directly associated to their poor cellular uptake as reported in Figure 2B&D for $P_{AW}$. In contrast when associated with Pep-1, these peptides (P1, P6, P10, P11) block viral proliferation with $IC_{50}$ values in the low $\mu M$ range, which correlated with their ability to inhibit HIV-1 RT *in vitro* (Table 4).

Table 4: Antiviral activity of Pep-A, P1 and P1-derived peptides

| Peptides | $EC_{50}$ (nM) | SI |
|---|---|---|
| P1/Pep-1 | 78.2 | >ND |
| P6/Pep-1 | 170 | > 3 |
| P8/Pep-1 | 290 | > 3 |
| P10/Pep-1 | 140 | ND |
| $P_{AW}$ | > 1000 | ND |
| $P_{AW}$/Pep-1 | 1.8 | > 550 |
| P18/Pep-1 | > 1000 | ND |
| P24/Pep-1 | 2.3 | > 1200 |
| P26/Pep-1 | > 1000 | ND |
| P27 | > 1000 | ND |
| P27/Pep-1 | < 0.32 | > 3100 |

ND: value not determined

In agreement with previous findings no antiviral activity was observed with Pep-A when associated to Pep-1 (Morris *et al.* 1999a). When complexed with Peep-1, $P_{AW}$ exhibits a marked antiviral activity with an $EC_{50}$ of 1.8 nM and a therapeutic/selectivity index of about 550. The 44- and 161-fold greater potency of $P_{AW}$ over peptides harbouring mutations at Glu[7] (P8) or lacking Trp residues (P1) confirms the requirement of these residues for targeting reverse transcriptase both *in vitro* and *in cellulo*. $P_{AW}$ constitutes a powerful inhibitor of polymerase activity and possesses a very potent antiviral activity without any toxic effect.

The 5 last residues at the C-terminus of $P_{AW}$ can be removed without affecting its potency to block viral replication (P24 : $EC_{50}$ = 2.3 nM). Interestingly, removing the last three residues of $P_{AW}$ is also associated with an increase in its antiviral activity with an $IC_{50}$ lower than 0.32 nM and a therapeutic/selectivity index greater than 3100 (P27).

**EXAMPLE II: ANALYSIS OF THE INTERACTION BETWEEN $P_{AW}$ PEPTIDE (SEQ ID NO: 18) AND HIV-1 REVERSE TRANSCRIPTASE**

**II.1. MATERIALS AND METHODS**

**Materials**

Primer and template oligonucleotides were from MWG Biotech AG, (Ebersberg, Germany). A 19/36-mer DNA/DNA primer/template was used for steady-state fluorescence titration and stopped-flow experiments, with 5'-TCCCTGTTCGGGCGCCACT-3' (SEQ ID NO: 30) for the primer strand and 5'-TGTGGAAAATCTCATGCAGTGGCGCCCGAACAGGGA-3' (SEQ ID NO: 31) for a template-strand. The sequence of the template strand corresponds to the sequence of the natural primer binding site (PBS) of HIV-1 (Wain-Hobson *et al.,* 1985). The primer was labelled at the 3'-end with 6-carboxyfluorescein (6-FAM) on thymine base. Primer and template oligodeoxynucleotides were separately resuspended in water and diluted to 100 $\mu M$ in annealing buffer (25 mM Tris pH 7.5 and 50 mM NaCl). Oligonucleotides were mixed together and heated at 95°C for 3 min, then cooled to room temperature for 1h.

**Steady-State Fluorescence Experiments**

Fluorescence experiments were performed in buffer containing 50 mM Tris-HCl, pH 8.0, 50 mM KCl, 10 mM $MgCl_2$ and 1 mM DTT, at 25°C, using a SPEX-PTI spectrofluorimeter in a 1 cm path-length quartz cuvette, with a band-pass of 2 nm for excitation and emission, respectively. Excitation was performed at 492 nm and emission spectra were recorded from 500 to 600 nm. According fluorescence experiments, a fixed concentration of FAM-labelled (19/36) p/t (50 nM) or of FITC-$P_{AW}$ (200 nM) was titrated with increasing protein concentrations from 5 nM to 1 $\mu M$. Data were fitted as described in Agopian *et al.* (2007) and Rittinger *et al.* (1995), using a quadratic equation (GraFit, Erithacus Software).

**HPLC Size Exclusion Chromatography**

Chromatography was performed using one (Phenomenex S3000) or two HPLC columns in series (Phenomenex S3000 followed by Phenomenex S2000; both 7.5 mm x 300 mm). Samples containing 3 to 10 μM of RT or p51 were applied onto one or two HLPC columns and eluted with 200 mM potassium phosphate (pH: 7.0) at a flow rate of 0.5 ml.min$^{-1}$ (5).

**Rapid kinetic experiments**

Binding kinetics of primer-template (p/t) onto HIV-1 RT were performed with a FAM-labelled p/t in buffer containing 50 mM Tris-HCl, pH 8.0, 50 mM KCl, 10 mM MgCl$_2$ and 1 mM DTT, using a stopped-flow apparatus (Hi-Tech Scientific, Salisbury, England-UK) at 25°C. A fixed concentration of FAM-labeled p/t (20 nM) was rapidly mixed with increasing concentrations of reverse transcriptase (RT) or RT/P$_{AW}$ complex formed at a 1/20 molar ratio (25 to 400 nM). 6-FAM-fluorescence was excited at 492 nm and emission detected through a filter with a cut-off at 530 nm. Data acquisition and analysis were performed using KinetAsyst 3 software (Hi-Tech Scientific, Salisbury, England-UK) and traces were fitted according to a three exponential equation, as previously described in Agopian *et al.* (2007). The rate constant for the first phase (k$_{+1}$ and k$_{-1}$), corresponding to the formation of a RT/P$_{AW}$-p/t collision complex, was extrapolated from the slope and the intercept with the y axis of the plot of k$_{obs1}$ vs RT concentrations. The k$_2$ (k$_{+2}$ + k$_{-2}$) and k$_3$ (k$_{+3}$ + k$_{-3}$) rate constants for the second and third phases corresponding to conformational changes of preformed RT/P$_{AW}$-p/t complex were directly obtained from the three exponential fitting.

Dissociation kinetics of HIV-1 RT were monitored by using 4,4'-Bis(1-anilinonaphthalene 8-sulfonate (bis-ANS) as extrinsic probe. Changes in bis-ANS fluorescence provide a good signal to probe variation in the exposure of the hydrophobic regions associated to RT dissociation in a time-dependent manner. 0.5μM of RT was dissociated in presence of 0.8 μM of bis-ANS, by adding 10 % acetonitrile in the absence or in the presence of 10 μM P$_{AW}$. Kinetics of dissociation were monitored by following fluorescence resonance energy transfer between tryptophan residues of RT and bis-ANS. Excitation of RT-Trp residues was performed at 290 nm and the increase of bis-ANS fluorescence emission at 490 nm was detected through a 420 nm cut-off filter. Data acquisition and analysis were performed using KinetAsyst 3 software (Hi-Tech Scientific, Salisbury, England-UK) and traces were fitted according to a single exponential equation.

**Cell Culture, transfection and indirect immunofluorescence microscopy**

HeLa cells were cultured in Dulbecco's modified Eagle's medium (DMEM) supplemented with 10% fetal calf serum at 37°C in a humidified atmosphere containing 5% CO$_2$. ). H9 cell lines (ATCC number: HTB-176) were stably transfected with pNL4.3 V-R+ plasmid (AIDS Research Reference Reagent Program, National Institutes of Health (NIH), USA) and therefore expressed in a constitutive manner all HIV-1 proteins but Env encoded proteins. They were cultured in RPMI-1640 medium supplemented with 2mM glutamine, 10% (w/v) foetal calf serum (FCS), 1% antibiotics (streptomycin 10 000 mg/ml, penicillin, 10 000 IU/ml), as well as G418 1 mg/ml. Cells were grown on glass coverslips to 75% confluency, then transfected with pcDNA3-p66RT plasmid (this plasmid contains the 66 subunit of HIV-1 Reverse Transcriptase cloned in pcDNA3.1 (+) vector (Invitrogen, USA), using *Hind*III and *Bam*HI restriction site) using Lipofectamine 2000 reagent according to manufacturer'instructions (Invitrogen). Hela cells were subsequently cultured for 32h, before incubation with FITC-P$_{AW}$ or FITC-P$_{AW}$/Pep-1 (complex obtained at a molar ratio 1/10) for 1 h. Coverslips were extensively rinsed with PBS and cells were fixed in 4% paraformaldehyde for 10 min and permeabilized in 0.2% Triton. After saturation in PBS supplemented with bovine serum albumine 1% for 1h, cells were incubated overnight with monoclonal 8C4 anti-HIV-1 RT antibody (AIDS Research Reference Reagent Program, NIH) (diluted 1:100 in PBS-BSA 1%), followed by Alexa-555 anti-mouse (Molecular Probes).

Immunofluorescence detection of HIV-1 RT and FITC-P$_{AW}$ was performed by epifluorescence microscopy using a PL APO 1.4 oil PH3 objective on a LEICA DMRA 1999 microscope. 3D reconstitution of the 20 frames realized from z stacking was performed using Imaris 6.0 software.

For RT pull down experiments, P$_{AW}$ (SEQ ID NO: 18) was incubated with 500 μl of activated CNBr-activated Sepharose 4B beads at 4°C overnight. After centrifugation, supernatants were removed and the beads were incubated with Glycine pH.8.0 for 2 hrs at 4°C with gentle stirring. The beads were then washed with 0.1 M sodium acetate buffer (pH 4.0), then 0.5 M Bicarbonate buffer, and finally in PBS, three times each. The peptide bound to the beads were then saturated for 30 min in PBS/BSA 0.1% and then incubated for 1h at 4°C with equal amounts of H9 cells lysed for 30 min on ice in Lysis buffer (Tris 20 mM, pH 7.2, NaCl 400 mM, EDTA 1mM, DTT 1mM, Protease inhibitors EDTA free) and sonicated 2 x 5 sec. at 20%. Beads were washed with Lysis buffer then twice with PBS and the bound proteins were finally separated on 15% SDS-PAGE gel and analyzed by Western Blotting using monoclonal 8C4 anti-HIV-1 RT antibody.

**II.2. RESULTS**

**$P_{AW}$ peptide interacts with HIV-1 RT in a cellular context**

The ability of $P_{AW}$ (SEQ ID NO: 18) to form stable complexes with HIV-1 RT expressed in cells was investigated by pull-down experiments. The peptides $P_{AW}$ and P8 (SEQ ID NO: 8) covalently associated with CNBr sepharose beads, was incubated in the presence of cell lysates of H9 cells expressing Gag-Pol gene products of HIV-1. Analysis of the presence of reverse transcriptiase (RT) by Western blotting revealed than only $P_{AW}$ was able to form stable complex with RT in a cellular context and to retain RT on beads (Figure 2A). In contrast, no RT was associated to free or P8-beads. To confirm that $P_{AW}$ targets HIV-1 RT in a cellular context, it has been further investigated its localization in cells transfected by a plasmid expressing HIV-1 p66 (pcDNA3-p66RT). As shown in Figure 2B, fluorescently-labelled peptide (FITC-$P_{AW}$) poorly enters cultured HeLa cells. In contrast, when complexed at a molar 1/10 ratio with the Pep-1 peptide-based nanoparticle delivery system, FITC-$P_{AW}$ rapidly (less than 1h) enters cells (Figure 2D). $P_{AW}$ localizes mainly in the cytoplasm with a peri-nuclear accumulation and partially colocalizes with HIV-1 RT as determined by indirect immunofluorescence (Figures 2E and 2G). The RT/$P_{AW}$ interaction in *cellulo* was further characterized using 3D reconstitution of frames from z stacking. 3D image analysis reveals that $P_{AW}$ does not enter the nucleus and co-localize with RT at the periphery of the nucleus (Figures 2G and 2H).

**Binding of $P_{AW}$ peptide to the dimeric form of HIV-1 RT**

To further understand the mechanism through which $P_{AW}$ inhibits RT, it has been investigated its potency to interact with the dimeric form of HIV-1 RT in the absence or presence of DNA/DNA p/t. The binding of $P_{AW}$ to RT was monitored using a fluorescently labelled peptide (FITC-$P_{AW}$). It has been first evaluated the impact of $P_{AW}$ labelling on the C-terminal cysteine with an FITC-probe on its ability to inhibit RT polymerase activity. As reported in Table 2 above, FITC-$P_{AW}$ blocks RT polymerase activity with a Ki of 2.7 $\pm$ 0.7 $\mu$M, 3.8-fold greater than for $P_{AW}$, suggesting that labelling has only a minor effect on $P_{AW}$ inhibitory property. As reported in Figure 3A, upon binding to the dimeric form of RT, the fluorescence of FITC-$P_{AW}$ was quenched by 39 % and analysis of the titration curves revealed that $P_{AW}$ tightly binds heterodimeric RT with a dissociation constant (Kd) of 33 $\pm$ 10 nM. When RT is first incubated with DNA/DNA p/t (18/36 mer), the quenching of FITC-$P_{AW}$ fluorescence associated with its binding was of 57% and the affinity of FITC-$P_{AW}$ for RT increased 5-fold (Kd : 7.1 $\pm$ 2.8 nM), suggesting that the presence of p/t on RT facilitates the binding of $P_{AW}$. The association of unlabelled $P_{AW}$ to RT was also evaluated by monitoring changes in fluorescently labelled p/t bound to RT (Figure 3B). Binding of $P_{AW}$ results in a 39 % quenching of fluorescence and a Kd value of 40 $\pm$ 18 nM was estimated from the titration binding curve. The 5.6-fold lower Kd of labelled FITC-$P_{AW}$ over unlabelled peptide, suggests that the dye contacts RT and stabilizes the peptide within its binding site.

As both Trp[24] and Phe[61] located on the fingers domain of p66 subunit have been reported to be involved in the control of p/t binding and in the dynamics of the thumb-fingers subdomain interactions (Agopian *et al.,* 2007 and Fisher *et al.,* 2002 and 2003), the binding of $P_{AW}$ on RT harbouring single Phe[61Gly] and double Phe[61Gly] and Trp[24Gly] mutations on the p66 subunit were then evaluated. In comparison to wild type RT, the affinity of $P_{AW}$ was reduced 6-fold (Kd : 207 $\pm$ 62 nM) for p66[F61G]/p51[wt] and 4.5-fold (Kd : 149 $\pm$ 38 nM) for p66[DM]/p51[wt] (Figure 3A).

**Effect of $P_{AW}$ peptide on primer/template binding to HIV-1 RT**

The impact of $P_{AW}$ peptide on the ability of HIV-1 RT to bind p/t was then investigated at both steady-state and pre-steady state levels using a 19/36 mer p/t labelled at the 3'-end of the primer with FAM-derivative as previously described (Agopian *et al.,* 2007 and Rittinger *et al.,* 1995). Results are shown in Figure 4A. The presence of a saturating concentration of $P_{AW}$ (10 $\mu$M) decreases the affinity of fluorescently labelled-p/t for RT 4.5-fold with a Kd value of 99 $\pm$ 40 nM in comparison to 22 $\pm$ 5 nM obtained in the absence of $P_{AW}$. The binding of unlabeled p/t induces a 42 % change in the fluorescence of $P_{AW}$-FITC pre-bound to RT and leads to a similar Kd value of 66.5 $\pm$ 19 nM (Figure 4A). These results suggest that p/t interacts close to $P_{AW}$ binding site on RT, inducing a change in the orientation of FITC linked to $P_{AW}$, but does not share the same binding site as already reported for Pep-A (Morris *et al.,* 1999a).

RT-p/t pre-steady-state binding kinetics follow a three-step mechanism in the presence or in the absence of $P_{AW}$, including a rapid diffusion controlled second order step leading to the formation of the RT-p/t collision complex, followed by two slow, concentration-independent, conformational changes (Agopian *et al.,* 2007). The plot of the pseudo-first order rate constant for the initial association of the p/t with RT against RT- concentration is linear. In the absence of $P_{AW}$, $k_{+1}$ and $k_{-1}$ rate constant values of $4.23 \cdot 10^8$ $M^{-1} \cdot s^{-1}$ and 29.9 $s^{-1}$ were calculated from the slope and the intercept with the y axis of the graph (Figures 4B and 4C). Analysis of the second and third slow phases yielded rate constants of $k_2 = 5.8$ $S^{-1}$ and $k_3 = 0.76$ $s^{-1}$ for RT. The presence of $P_{AW}$ does not alter the overall Kd1 for the initial formation of the RT-p/t complex as both the "on" ($k_{+1} = 1.05 \cdot 10^8$ $M^{-1} \cdot s^{-1}$) and the "off" ($k_{-1} = 7.9$ $s^{-1}$) rates of the first step are decreased by about 4-fold. In contrast, the presence of $P_{AW}$ on RT significantly reduced the rate constants of the slow conformational steps ($k_2$ =1.99 s$^{-1}$ and $k_3$ = 0.22 s$^{-1}$), affecting the proper binding of the p/t (Figure 4B & 4C).

### Effect of P$_{AW}$ on the stability and dimerization of HIV-1 RT

The impact of $P_{AW}$ on the stability and formation of heterodimeric-RT was investigated by size-exclusion chromatography as previously described (Divita *et al.,* 1995a). Results are represented in Figure 5A. Heterodimeric-RT incubated or not in the presence of an excess of $P_{AW}$ (100μM) for 1h30 at room temperature, is fully dimeric and eluted as a single peak at 16.7 min. The interaction of $P_{AW}$ with RT was monitored by size-exclusion chromatography using HIV-1 RT pre-incubated with FITC-$P_{AW}$. Chromatography analysis reveals that FITC-$P_{AW}$ co-elutes with heterodimeric RT in a single peak at 16.7 min (Figure 5A), demonstrating that $P_{AW}$ binds heterodimeric RT and does not induce RT dissociation. The ability of $P_{AW}$ to interact with p66 or p51 monomeric forms was then evaluated. Experiments performed with a partially dissociated RT/$P_{AW}$ (50%) complex by 10% acetonitrile, show that $P_{AW}$ remains associated only with the dimeric fraction of RT, and does not bind monomeric p66 or p51 subunits, which are eluted at 17.5 min and 18.2 min, respectively (Figure 5B).

The ability of $P_{AW}$ to prevent HIV-1 RT-dimerization was then investigated. Dissociation of RT was achieved at room temperature with 17% acetonitrile, and then association of the subunits was induced by a 10-fold dilution of the sample in an acetonitrile-free buffer in the absence or presence of 100 μM of $P_{AW}$. At this concentration (1.7%) of acetonitrile no dissociation of RT could be detected. As shown in Figure 6A, heterodimeric RT was fully re-associated 5 hrs after dilution in an acetonitrile-free buffer, both in the absence or in the presence of $P_{AW}$ (100 μM), indicating that $P_{AW}$ does not block RT dimerization (Figure 6A). The impact of $P_{AW}$ was further investigated on the kinetics of RT-dimerization. The level of dimeric RT was evaluated 30 min and 2 hrs after dilution in free acetonitrile buffer by size-exclusion chromatography (Figures 6B and 6C). In the presence of $P_{AW}$ 21 % and 59 % of dimeric RT was quantified after 30 min and 2 hrs respectively (Figure 6B). In comparison only 16 % (30 min) and 29 % (2hrs) of dimeric RT were detected in the absence of peptide (Figure 6C), suggesting that the presence of $P_{AW}$ favours the kinetics of RT dimerization.

### P$_{AW}$ peptide favours dimerization of the small p51 subunit

P51 subunits are mainly monomeric and dissociation constants for p51/p51 homodimer have been reported to be either in the μM (Venezia *et al.,* 2006) or mM (Restle *et al.,* 1990) range depending on the technology used to quantify the interactions. The ability of $P_{AW}$ to favour p51/p51 dimerization has been investigated by size exclusion chromatography, using two HPLC columns in series. Experiments were performed at a p51 concentration of 3.5 μM at which it is entirely monomeric and elutes as a single peak at 32.7 min. Monomeric p51 (3.5μM) was incubated in the presence of FITC-labelled $P_{AW}$ (20 μM) for 1 h at room temperature then analysed by size exclusion chromatography. Results are shown in Figure 7. In the presence of fluorescently-labelled $P_{AW}$ 4.6 % of p51 are dimeric and associated to $P_{AW}$, suggesting that $P_{AW}$ promotes p51/p51 homodimer and only p51/p51 homodimer.

### P$_{AW}$ eptide prevents HIV-1 RT dissociation

The impact of $P_{AW}$ on HIV-1 RT stability and dissociation were investigated at the steady state level by size exclusion chromatography and at the pre-steady state level by stopped-flow rapid kinetics. HIV-1 RT was preincubated in the presence of 100 μM $P_{AW}$, for 2 hrs, prior dissociation with 17% or 10 % of acetonitrile, and the level of dimeric form was then assessed by size exclusion chromatography and the rate of dissociation by pre-steady-state kinetics. As reported in Figure 8A, the presence of $P_{AW}$ protects RT from the acetonitrile dissociation as 17% remains dimeric whereas "free" RT is completely dissociated with 17% acetonitrile.

The protection by $P_{AW}$ of acetonitrile-associated RT-dissociation was further investigated by monitoring pre-steady-state dissociation kinetics of HIV-1 RT, using bis-ANS as an extrinsic probe (Divita *et al.,* 1995c). Binding of bis-ANS to dissociated RT resulted in a large increase in the fluorescence of the probe due to non covalent interactions of bis-ANS to exposed hydrophobic surfaces on RT subunits, therefore providing a good signal for following RT dissociation in a time-dependent manner. Experiments were performed by adding bis-ANS to HIV-1 RT prior dissociation of the enzyme by 10% acetonitrile and monitoring FRET between exposed Trp of RT and Bis-ANS. As reported in Figure 8B, the kinetics of increased ANS-fluorescence upon dissociation of RT in the absence of $P_{AW}$ follow a single-exponential reaction, with a dissociation rate constant $k_{dis}$ of 5.30 $\pm$ 0.01 s$^{-1}$, which is reduced 3.8-fold ($k_{dis}$ =1.42 $\pm$ 0.007 s$^{-1}$), when RT is incubated with $P_{AW}$.

**EXAMPLE III: ANALYSIS OF THE INTERACTION BETWEEN P$_{AW}$ PEPTIDE (SEQ ID NO: 18) AND HIV-1 INTEGRASE (IN)**

**III.1. MATERIALS AND METHODS**

**Materials**

Peptides were prepared as described in Example I.1 above.

**Western Blot Analysis and Antibodies**

The protein-containing supernatants were separated by SDS/PAGE, transferred onto Nitrocellulose membranes, and revealed by immunoblotting with the following antibodies as indicated: polyclonal anti-Integrase, or monoclonal anti-HA (HA.11; Sigma).

**Cell culture**

Adherent fibroblastic HeLa cell line stably expressing HA-tagged Integrase (HeIIN cells) (Mousnier *et al.,* 2007) were cultured in DMEM supplemented with 2mM glutamine, 1% antibiotics (streptomycin 10 000 mg/ml, penicillin, 10 000 IU/ml), 10% (w/v) foetal calf serum (FCS), and 1 µg/ml Puromycin (SIGMA). Modified ATCC H9 cell lines were cultured and prepared as described in Example II.1 All cells lines were maintained at 37°C in a humidified atmosphere containing 5% CO2.

**Integrase pull downs using CNBR Sepharose activated beads**

Peptides were resuspended in buffer A (1 mg/ml), sonicated 4 min, and incubated with 500 µl (gel volume) of activated CNBr-activated Sepharose 4B sepharose© beads at 4°C overnight. After centrifugation, supernatants were removed and the beads were incubated with Glycine pH.8 for 2 hours at 4°C with gentle stirring. The sedimented Sepharose beads were then washed in 0.1 M Sodium Acetate buffer (pH 4), 0.5 M Bicarbonate buffer, and finally in PBS, three times each. The peptides bound to the beads were then saturated for 30 minutes in PBS BSA 0.1% and then incubated for 1h at 4°C with equal amounts of cell lysate (H9 or Hela-IN) prealably lysed for 30 min on ice in Lysis buffer (Tris 20 mM, pH 7,2, NaCl 400 mM, EDTA 1mM, DTT 1mM, Protease inhibitors EDTA free (Roche Diagnostics) and sonicated twice for 5 sec. at 20%. Beads were washed once in Lysis buffer, and twice in PBS. They were finally resuspended in Laemli blue, migrated on 15% SDS-PAGE gels and analysed by Western Blotting.

**Cellular localization experiments and Pep-1-mediated transfection**

For carrier peptide Pep-1 (SEQ ID NO: 29) mediated delivery of P$_{AW}$ peptide, stock solutions of Pep-1-P$_{AW}$ complex were formed by incubation of P$_{AW}$ (SEQ ID NO: 18) with the carrier peptide at a molecular ratio of 1/10 in PBS for 30 min at 37°C. They were then diluted in DMEM to the desired concentration.
Cells were grown on acid-treated glass coverslips to 60% confluence. Once rinsed with PBS, cells were overlaid with preformed complexes and incubated for 40 minutes at 37°C. Cells were then rinsed twice with PBS and fixed 15 min at Room Temperature (RT°) in Paraformaldehyde 4%, and washed three times in PBS + 2% BSA. They were then permeabilised by incubation 10 min RT° in PBS+ 0.5% Triton X-100, washed again 3 times in PBS+ 2% BSA. After blocking 20 min in the same solution, the coverslips were placed in a humid chamber, overlaid with 75 µl of primary antibody against Integrase, and incubated for 2h at 37°C. Coverslips were then rinced once with PBS and incubated with 75 µl of anti rabbit antibody diluted 1/10000 in PBS. After immobilisation on the cover using Prolong Gold antifade reagent, cellular localization of Integrase as well as FITC-labelled peptides was monitored by fluorescence microscopy, using a PL APO 1.4 oil PH3 objective on a LEICA DMRA 1999 microscope. For suspension cell lines, cells were harvested by centrifugation and resuspended directly with the preformed complex solutions for 5 min and then the level of foetal calf serum was adjusted to 10%.

**Cycloheximide treatment**

Cycloheximide treatment was done as described in Mousnier *et al.* (2007). Cells were incubated at 37° C with 100 mg/ml cycloheximide for various periods of time prior to washing in PBS and lysis in Laemmli sample buffer. Protein content of the total cell lysates was quantified (Bio-Rad protein assay kit). Equal amounts of total cellular proteins were resolved by SDS/PAGE and analyzed by Western blot with the indicated antibodies.

## Fluorescence anisotropy experiments

Steady-state fluorescence anisotropy parameter (r) was recorded on a Beacon 2000 instrument (PanVera, Madison, USA), in a cell thermostatically held at 25°C for DNA-binding assay or 37°C for activity test (the sample volume was typically 200 μL).

## Integrase-peptide interaction

Fluorescein-labeled peptides (40 nM) were mixed with varying concentrations of integrase in 20 mM Tris pH 7.2, 50 mM NaCl, 10 mM $MgCl_2$, and the r values were recorded. Fluorescence intensities of peptides did not significantly changed upon addition of integrase.

## DNA-binding of integrase and 3'-processing assay

The simultaneous measurement of integrase-DNA interaction and subsequent 3'-processing activity by steady-state fluorescence anisotropy was performed as described in Guiot *et al.* (2006). 4 nM of fluorescein-labeled double stranded oligonucleotide (21-mer oligonucleotide mimicking the U5 LTR extremity with fluorescein attached on the 3'-extremity of the processed end) was added to a preincubated mixture containing IN (100 nM) and increasing concentration of unlabeled peptides in a Tris buffer (20 mM, pH 7.2) containing 50 mM NaCl, 10 mM $MgCl_2$ and 1 mM DTT. r values obtained upon addition of integrase ($r_{complex}$) were used to measure the IN-DNA interaction as IN binding to the fluorescein-labeled oligonucleotide significantly increases the fluorescence anisotropy. After the initial DNA-binding step (performed at 25°C), the 3'-processing activity was measured at 37°C by fixed-time experiments: The reaction was stopped at varying times by adding SDS (0.25% final) which disrupts all IN-DNA complexes in the sample. The IN-mediated cleavage of the terminal GT dinucleotide (labeled by fluorescein on the 3'-end) leads to a significant decrease of r as compared to the r value corresponding to the fluorescein-labeled unprocessed DNA substrate. The fraction of released dinucleotide is calculated by using Eq. (1):

$$F_{dinu} = \frac{r_{NP} - r}{r_{NP} - r_{dinu}} \qquad (1)$$

wherein $r_{Np}$ and $r_{dinu}$ are the anisotropy values corresponding to the unprocessed double-stranded DNA substrate and GT dinucleotide, respectively.

For dissociation experiments, IN-DNA substrate complexes were preformed at 25°C before addition of peptides. The peptide-mediated dissociation of complexes was then studied at the same temperature by measuring the decrease in the r value as a function of time.

### III.2. RESULTS

## $P_{AW}$ peptide interacts with HIV-1 integrase

$P_{AW}$ peptide (SEQ ID NO: 18) was first assessed on its ability to form stable complex with HIV-1 integrase either recombinant or expressed in cells, by pull-down and steady-state fluorescence anisotropy experiments. $P_{AW}$ covalently associated to CNBr sepharose beads, was incubated with either recombinant IN or cell lysate of H9 and Hela cells, expressing GAG-POL gene products of HIV-1, or HA-tagged IN, respectively. Beads were then extensively rinsed and presence of IN was detected by western blotting.

$P_{AW}$ was able to interact with cellular HA-tagged IN. $P_{AW}$ beads retained cellular IN when expressed at low level in H9 cells. As a control, the reverse transcriptase (RT) dimerization inhibitor Pep-7 (Morris *et al.,* 1999b) does not bind IN, suggesting a specific impact of the Trp residues in the $P_{AW}$ context.

The direct interaction between $P_{AW}$ and integrase was further assessed by steady-state fluorescence anisotropy using fluorescein-labeled peptides. The steady-state anisotropy value (r) of labelled-$P_{AW}$ was measured in the presence of increasing concentrations of IN. Upon addition of integrase, the r value of $P_{AW}$ increased (Δr = 0.060). Fitting of the titration binding curve leads to an apparent dissociation constant for the $P_{AW}$ /IN complex of $Kd_{app} \approx 400$ nM in perfect agreement with the tight $P_{AW}$ /IN interaction obtained in the pull down experiments. The impact of the $P_{AW}$ peptide onto IN oligomerization was also assessed as described in Deprez *et al.* (2001) and no effect of $P_{AW}$ on the IN oligomer integrity was observed.

**$P_{AW}$ peptide inhibits IN 3' processing activity in vitro**

The potency of $P_{AW}$ to inhibit 3' processing activity of IN was evaluated using a steady-state fluorescence anisotropy *in vitro* assay as described in Guiot *et al.* (2006). A fluorescently labelled DNA was used to monitor the binding of IN to its DNA substrate and the subsequent 3' processing activity, both event being associated with changes on the anisotropy parameters.

It was first validated that $P_{AW}$ does not interact with DNA, then the 3'-processing kinetics of IN were measured in the absence or presence of a fixed $P_{AW}$ concentration of 100 $\mu$M. As reported in Figure 9A, the 3'-processing activity was totally abolished with 100 $\mu$M $P_{AW}$, suggesting a correlation between the propensity of $P_{AW}$ to interact with IN and its inhibitory effect on the catalytic activity. The $P_{AW}$ mediated inhibition of the 3'-processing activity is peptide concentration dependent and an $IC50_{3'\text{-}proc}$ value of 12.7 $\mu$M was calculated (Figure 9B).

Results were identical when $P_{AW}$ peptide further contained a cysteine residue at the C-terminus.

**$P_{AW}$ induces dissociation of preformed IN/DNA complexes**

To get insight into the mechanism of inhibition, the ability of $P_{AW}$ to prevent IN-DNA recognition was measured. As reported in Figure 9C, the number of IN/DNA complexes decreased as a function of $P_{AW}$ concentration, with an $IC50_{DNA\text{-}binding}$ value of 15.2 $\mu$M, compatible with the value found for the inhibition of the 3' processing activity. This suggests that $P_{AW}$ does not inhibit the catalytic process by itself but most likely inhibits the DNA-binding step as previously reported for small chemical compounds such as styrylquinoline derivatives (Deprez *et al.,* 2004). However, in contrast to styryquinoline compounds which were not active on preformed IN-DNA complexes (Deprez *et al.,* 2004), $P_{AW}$ does not prevent association of IN to its DNA substrate, but also effectively dissociates preformed complexes in a concentration dependent manner with similar efficiency than the one obtained when $P_{AW}$ and IN were pre-incubated before adding the DNA substrate (Figure 9D). Interestingly, the apparent dissociation constant measured for the formation of $P_{AW}$-IN complex (400 nM) was significantly below the $IC50_{3'P}$ and $IC50_{DNA\text{-}binding}$ values. Two alternative hypothesis can account for this apparent discrepancy: (i) as the IN/$P_{AW}$ interaction was probed using a fluorescein-labelled counterpart of $P_{AW}$, it could be possible that the fluorescein moiety stabilizes the complex, (ii) the high affinity site is not directly related to the competitive inhibition mode.

In order to verify the influence of the fluorescent probe attached to $P_{AW}$ on the peptide-integrase interaction, a standard gel-electrophoresis procedure (as described in Smolov *et al.,* 2006) was carried out to examine the IN 3'-processing activity as a function of either unlabeled or fluorescein-labeled $P_{AW}$ concentration. The fluorescein-labeled $P_{AW}$ was characterized by an $IC50_{3'\text{-}proc}$ value of 10 $\mu$M, in the same range that the value found for the unlabeled $P_{AW}$, suggesting that the fluorescein does not influence the binding or inhibition properties of $P_{AW}$. Thus, the discrepancy between the apparent affinity for the IN-$P_{AW}$ complex (submicromolar) as compared to the $IC_{50}$ value (in the 10-15 $\mu$M range) most likely accounts for the presence of peptide binding site on the integrase that differs from its active site and indirectly influence 3'-processing activity.

**Insight into the cellular mechanism of $P_{AW}$**

The fact that $P_{AW}$ blocks viral production with a subnamolar $EC_{50}$ cannot only be explained by inhibition of IN enzymatic activity ($IC50_{3'\text{-}proc}$: 15.2 $\mu$M) and is more correlated to the tight binding of the peptide to IN. In order to address that point, it has been investigated the mechanism by which this peptide alters IN activities at the cellular level.

Hela cells stably expressing Ha-tagged IN were used to analyze the cellular behaviour of IN on $P_{AW}$-treated cells. As previously reported, the PIC-mediated nuclear import of IN constitutes a major step in the infection cycle and IN mainly localizes in the nucleus (Bukrinsky *et al.,* 1992 ; Depienne *et al.,* 2001 and Piller *et al.,* 2003). As reported in Figure 10, in the absence of $P_{AW}$, Ha-tagged IN localizes in the nucleus of Hela cells. In contrast, when cells were treated with $P_{AW}$ (1 $\mu$M), IN did not localizes in the nucleus, suggesting that IN interaction with $P_{AW}$ within the cell, prevents its nuclear localization and/or induces its accumulation/retention in the cytoplasm. The impact of $P_{AW}$ has been further investigated in High Content Screening (HCS) using a higher number of cells. Statistic analysis of the cytoplasmic retention of IN has revealed that in the absence of $P_{AW}$ more than 90% of the integrase located in the nucleus, in contrast to only 35% when cell are treated with $P_{AW}$.

It has also been demonstrated that the cytoplasm retention of IN is dependent on the concentration of $P_{AW}$ used and does not occur with either the free Pep-1 (SEQ ID NO: 29) particles or P8 peptide (SEQ ID NO: 8)/Pep-1 complexes (Figure 10B).

**$P_{AW}$ alters the stability of IN**

In the presence of $P_{AW}$, IN was mostly detected in the cytoplasm. It has been investigated to what extend $P_{AW}$

was acting on pre-existing integrase or rather that on newly-formed protein.

It was previously shown that HA-tagged IN is a very unstable protein when overexpressed in Hela cells, with a half life of 23 min, as estimated by cycloheximide treatment (Mousnier *et al.,* 2007). Therefore the stability of IN in the presence of $P_{AW}$ was investigated. Cells were treated with Cycloheximide for 0, 30 or 90 minutes, in the absence of the presence of 1 μM of $P_{AW}$ (Figure 10D). In the absence of $P_{AW}$, IN a half-life was estimated at about 30 min, which is consistent with the results previously described (Mousnier *et al.,* 2007). In contrast, in the presence of the peptide, IN half-life was reduced by about 2-fold, (estimated 17 min).

Taken altogether, these results suggest that $P_{AW}$ binding induces a structural destabilisation of IN, which alters interaction with partners and promotes its degradations, resulting in an apparent nuclear delocalisation.

## EXAMPLE IV: $P_{AW}$, P16 AND P27 BLOCK RESISTANT STRAINS AND HIV CLADES

### IV.1. MATERIALS AND METHODS

**Materials**

$P_{AW}$ (SEQ ID NO: 18), P16 (SEQ ID NO: 19), P27 (SEQ ID NO: 25) and Pep-1 (SEQ ID NO: 29) peptides were prepared as described in Example I.1 above. Pep-3 peptide (SEQ ID NO: 33) was prepared as described in Morris *et al.,* 2007. Pep-7 peptide (SEQ ID NO: 32) was prepared as described in Morris *et al.,* 1999b.

HIV strains BH10, 1650, RF, 2914, NDK, 2165, HIV-1 215Y, HIV-1 67N, 70R, 215F, 219Q, HIV-1 74V, HIV-1 N119/181C and HIV-1 41L, 74V, 106A, 215Y were obtained from the National Institutes of Health, USA (AIDS Research Reference Reagent Program).

**Methods**

The anti-HIV activities of the peptides were assayed according to the methods described in Roisin *et al.* (2004) (see also Example I.1 above).

IV.2. RESULTS

**$P_{AW}$ and P27 block replication of HIV-1 clades and combining Pep-7/P27 improves efficiency**

P27 peptide was associated to the peptide based nanoparticle delivery system (Pep-1 or Pep-3) at a ratio 20/1. For combining experiments, equi-molar concentrations of Pep-7 and P27 were associated with the carrier Pep-3 at a molar ratio of 1/20 and applied onto anti HIV evaluation. Results are shown in Table 5 below. Data are the averages of three separate experiments.

Table 5:

| HIV-1 Strain | Clade | Country | IC50 (nM) | | |
|---|---|---|---|---|---|
| | | | $P_{AW}$ | P27 | P27/Pep-7 |
| BH10 | | | 4.6 | 1.4 | 0.3 |
| 1650 | A | France | 4.1 | 0.8 | 0.8 |
| RF | B | Haiti/USA | 3.5 | 1.5 | 0.4 |
| 2914 | C | USA | 2.4 | 0.2 | 0.3 |
| NDK | D | Zaire | 1.5 | 0.3 | 0.09 |
| 2165 | E | Asia | 5.1 | 2.6 | 0.7 |

**$P_{AW}$ and P27 block replication of resistance strains and combining P71/P27 improves antiviral potency**

Results are shown in Table 6 below:

| RT Genotype | Phenotype | IC50 (nM) | | |
|---|---|---|---|---|
| | | P$_{AW}$ | P27 | P27/Pep-7 |
| HIV-1215Y | AZT-resistant | 4.1 | 2.7 | 2.1 |
| HIV-1 67N, 70R, 215F, 219Q | AZT-resistant | 3.7 | 1.5 | 0.9 |
| HIV-1 74V | resistant to ddI and ddC | 5.2 | 3.8 | 1.3 |
| HIV-1 N119/181C | resistant to nevapirine and non nucleoside RT inhibitors | 5.1 | 2.0 | 1.8 |
| HIV-1 41L, 74V, 106A, 215Y | resistant to AZT, ddI, nevapirine; non nucleoside RT inhibitors | 4.8 | 4.9 | 1.4 |

### P16 blocks replication of HIV-1clades and combining P16/Pep-7 improves efficiency

P16 peptide was associated to the peptide based nanoparticle delivery system (Pep-1 or Pep-3) at a ratio 20/1. For combining experiments, equimolar concentrations of Pep-7 and P16 were associated with the carrier Pep-3 at a molar ratio of 1/20 and applied onto anti HIV evaluation. Results are shown in Table 7 below. Data are the averages of three separate experiments.

Table 7:

| Strain | Clade | Country | IC50 (nM) | IC50 (nM) |
|---|---|---|---|---|
| | | | P16 | P16/Pep-7 |
| BH10 | A | France | 5.9 | 1.2 |
| 1650 | B | Haiti/USA | 7.5 | 0.5 |
| RF | C | USA | 10.9 | 1.9 |
| 2914 | D | Zaire | 1.7 | 2.0 |
| NDK | E | Asia | 2.9 | 0.2 |
| 2165 | F | France | 2.7 | 1.8 |

### P16 and P16/Pep-7 block replication of resistance strains

Results are shown in Table 8 below:

| RT Genotype | Phenotype | IC50 (nM) | |
|---|---|---|---|
| | | P27 | P27/Pep-7 |
| HIV-1215Y | AZT-resistant | 3.9 | 2.1 |
| HIV-1 67N, 70R, 215F, 219Q | AZT-resistant | 5.2 | 4.0 |
| HIV-1 74V | resistant to ddI and ddC | 4.9 | 2.6 |
| HIV-1 N 119/181 C | resistant to nevapirine and non nucleoside RT inhibitors | 6.3 | 3.1 |
| HIV-1 41L, 74V, 106A, 215Y | resistant to AZT, ddI, nevapirine; non nucleoside RT inhibitors | 10.5 | 1.9 |

### EXAMPLE V: P$_{AW}$ AND DERIVED-PEPTIDES THEREFROM ALTER IN STRUCTURE AND FUNCTION

### V.I. MATERIALS AND METHODS

### Materials

P$_{aw}$ (SEQ ID NO: 18) P16 (SEQ ID NO: 19), P17 (SEQ ID NO: 20), P18 (SEQ ID NO: 21), P24 (SEQ ID NO:

23), P26 (SEQ ID NO: 24) and P27 (SEQ ID NO: 25) were prepared as described in Example I.1 above.

## Methods

Effect of the different peptides on the integrase (IN) activity, DNA binding and IN/DNA dissociation were evaluated as described in Example III.1 above, using fluorescence polarization and anisotropy measurements. Binding constant of the peptide for IN was determined by steady state fluorescence and anisotropy.

Dissociation of IN/LEDGF complex was measured by fluorescence resonance energy transfer (AlphaScreen® technology) and immunoprecipitation experiments:

- Amplified Luminescent Proximity Homogeneous Assay (ALPHA): when the proteins (IN and LEDGF), linked to acceptor and donor beads, interact together (*i.e.,* are into close proximity), a photosignal (a high amplified signal with output in the 520-560 nm range) is detected; when the proteins are not in close proximity, the reactive oxygen decays and there is no detectable signal generated;
- immunoprecipitation (IP) was performed using polyclonal anti LEDGF

antibodies (purchased from Santa Cruz Biotechnology, USA) and polyclonal HA antibodies (purchased from Eurogentec, Belgium). Cell line expression HA-tagged integrase was using for the experiment in the presence of increasing concentrations of peptide inhibitors.

## V.2. RESULTS

Results are shown in Table 9 below:

| Peptides | Kd (nM) | EC50 ($\mu$M) | | | |
|---|---|---|---|---|---|
| | | 3'processing | IN/DNA binding | IN/DNA dissociation | IN/LEDGF dissociation |
| $P_{AW}$ | 230 nM | 12 $\mu$M | 10 $\mu$M | 15 $\mu$M | 2 $\mu$M |
| P16 | 120 nM | 10$\mu$M | 8 $\mu$M | 11 $\mu$M | 0.8 $\mu$M |
| P24 | 350 nM | 33 $\mu$M | 35 $\mu$M | 21 $\mu$M | 13 $\mu$M |
| P26 | 320 nM | 28 $\mu$M | 54 $\mu$M | 67 $\mu$M | >50 $\mu$M |
| P27 | >100 $\mu$M | > 100 $\mu$M | > 100 $\mu$M | > 100 $\mu$M | NO |
| P18 | NO | NO | NO | NO | NO |
| P17 | NO | NO | NO | NO | NO |

$P_{AW}$ and P16 tightly interacted with integrase and therefore induce conformational changes than prevent and even dissociate interactions between IN and its DNA substrate, and a cellular partner (LEDGF).

## EXAMPLE VI: EVALUATION OF CYCLIC $P_{AW}$, P16 AND P27 PEPTIDES

## VI.1. MATERIALS AND METHODS

## Materials

Peptide cyclization was performed via disulfide linkages S-S bond. $P_{AW}$ (SEQ ID NO: 18), P16 (SEQ ID NO: 19) and P27 (SEQ ID NO: 25) where respectively cyclised by either adding to the peptides sequences a cysteine residue at the N-terminus and a cysteine residue at the C-terminus, or by substituting the amino acid residue at position 2 by a cysteine residue and by adding a cysteine residue at the C-terminus:

Peptides sequences:

PAW-C1: CGTKWLTEWIPLTAEAEC (SEQ ID NO: 35)

PAW-C2: GCKWLTEWIPLTAEAEC (SEQ ID NO: 36)
P16C1: CGTKWLTEVWPLC (SEQ ID NO: 37)
P16C2: GCKWLTEVWPLC (SEQ ID NO: 38)
P27C1: CGTKWLTEWIPLTAEC (SEQ ID NO: 39)
P27C2: GCKWLTEWIPLTAEC (SEQ ID NO: 40)

## Methods

Effect of the different cyclized peptides on RT polymerase activity was determined as described in Example I above. The effects on IN activity, DNA binding and IN/DNA dissociation were evaluated as described in Example III above, using fluorescence polarization and anisotropy measurements. Binding constant of the peptide for IN was determined by steady state fluorescence and anisotropy.

### VI.2. RESULTS

Results are shown in Table 10 below:

| Peptides | Ki RT polymerase | Kd IN binding | Ki IN 3'processing | EC50 HIV-1 lai |
| --- | --- | --- | --- | --- |
| PAW | 0.7 $\mu$M | 230 nM | 12 $\mu$M | 1.8 nM |
| PAW-C1 | 0.2 $\mu$M | 290 nM | 21 $\mu$M | 1.2 nM |
| PAW-C2 | 1.4 $\mu$M | 120 nM | 8 $\mu$M | 2.7 nM |
| P16 | 14.4 $\mu$M | 120 nM | 10 $\mu$M | 5.9 nM |
| P16C1 | 32.1 $\mu$M | 90 nM | 4.3 $\mu$M | 2.5 nM |
| P27 | 0.05 $\mu$M | > 100 $\mu$M | > 100 $\mu$M | 0.21 nM |
| P27C1 | 0.042 $\mu$M | >100 $\mu$M | >100$\mu$M | 0.17 nM |

Cyclized $P_{AW}$, P 16 and P27 peptides respectively inhibit RT polymerase and IN 3' processing activity and alter the stability of IN *in vitro.*

## REFERENCES

Agopian, A. et al. (2007) J. Mol. Biol. 373, 127-140.
Anker, M. and Corales, R. B. (2008) Expert Opin. Investig. Drugs 17, 97-103 Barre-Sinoussi, F. et al. (1983) Science 220, 868-871
Bukrinsky, M. I. et al. (1992) Proc. Natl. Acad. Sci. U S A 89, 6580-6584
Cabodevilla, J. F. et al. (2001) Eur. J. Biochem. 268, 1163-1172
Camarasa, M. J. et al. (2006) Antiviral Res. 71, 260-267
Cherepanov, P. et al. (2003) J Biol. Chem. 278, 372-81
Craigie, R. (2001) J Biol. Chem. 276, 23213-6
De Clercq, E. (2007) Nat. Rev. Drug Discov. 6, 1001-1018
De Coupade, C. et al. (2005) Biochem. J. 390, 407-18
Depienne, C. et al. (2000) Experimental Cell Research 260, 387-395
Depienne, C. et al. (2001) J. Biol. Chem. 276, 18102-18107
Depollier, J. et al. (2005) Biochemistry 44, 1909-1918
Deprez, E. et al. (2001) Proc. Natl. Acad. Sci. U S A 98, 10090-10095.
Deprez, E. et al. (2004) Mol. Pharmacol. 65, 85-98
Derossi, D. et al. (1996) J Biol. Chem. 271, 18188-18193
Di Marzo Veronese, F. et al. (1986) Science 231, 1289-1291
Divita, G. et al. (1994) J. Biol. Chem. 269, 13080-13083
Divita, G. et al. (1995a) J. Biol. Chem. 270, 28642-28646
Divita, G. et al. (1995b) J. Mol. Biol. 245, 508-521

Divita, G. et al. (1995c) Biochemistry 34, 16337-16346

Dyda, F. et al. (1994) Science 266, 1981-6

Elliott, G. and O'Hare, P. (1997) Cell 88, 223-233

Farnet, C. M. et al. (1997) Cell 88, 483-92.

Figueiredo, A. et al. (2006) PLoS pathogens 2, e 119

Fisher, T. S., and Prasad, V. R. (2002) J. Biol. Chem. 277, 22345-22352

Fisher, T. S., Darden, T., and Prasad, V. R. (2003) J. Mol. Biol. 325, 443-459 Ghosh, M. et al. (1996) Biochemistry 35, 8553-8562

Grinsztejn, B. et al. (2007) Lancet 369, 1261-9

Gros, E. et al. (2006) Biochimic et biophysica acta 1758, 384-393

Guiot, E. et al. (2006) 281, J Biol. Chem. 281, 22707-22719

Hsiou, Y. et al. (1996) Structure 4, 853-860

Huang, H. et al. (1998) Science 282, 1669-1675

Huang, S. C. et al. (1992) Biochem. Biophys. Res. Commun. 184, 986-992

Jacobo-Molina, A. et al. (1993) Proc. Natl. Acad. Sci. USA 90, 6320-6324

Kärber. (1931) Naunyn-Schmiedebergs Archiv für experimentelle Pathologie und Pharmakologie 162, 480-483

Kohlstaedt, L. A. et al. (1992) Science 256, 1783-1790

Kokryakov, V .N. et al. (1993) FEBS Lett. 327, 231-236

Lightfoote, M. M. et al. (1986) J. Virol. 60, 771-775

Malet, I. et al. (2008) Agents Chemother. 52, 1351-1358

Matsuzaki, Y. et al. (2006) Presented at the XIIIth CROI Denver, USA, February 5-9 2006

Morris, M. C. et al. (1999a) Biochemistry 38, 15097-15103

Morris, M. C. et al. (1999b) J. Biol. Chem. 274, 24941-24946

Morris, M. C. et al. (2001) Nat.Biotech. 19, 1173-1176

Morris, M. C. et al. (2007) Nucleic Acids Res. 35, e49

Mossmann T. (1983) J. Immunol. Methods 65, 55-63

Mousnier, A. et al. (2007) Proc. Natl. Acad. Sci. U S A 104, 13615-20.

Muller, B. et al. (1989) J. Biol. Chem. 264, 13975-13978

Patel, P. H. et al. (1995) Biochemistry 34, 5351-5363

Piller, S. C. et al. (2003) Curr. Drug Targets 4, 409-29

Pommier, Y. et al. (2005) Nat. Rev. Drug Discov. 4, 236-48

Popov, S. et al. (1998) EMBO J. 17, 909-17

Restle, T. et al. (1990) J. Biol. Chem. 265, 8986-8988

Rittinger, K. et al. (1995) Proc. Natl. Acad. Sci. U S A 92, 8046-8049

Rodgers, D. W. et al. (1995) Proc. Natl. Acad. Sci. USA 92, 1222-1226

Roisin, A. et al. (2004) J. Biol. Chem. 279, 9208-9214

Ruben, S. et al. (1989) J. Virol. 63, 1-8

Sato, M. et al. (2006). J. Med. Chem. 49, 1506-1508

Semenova, E. A. et al. (2008) Adv. Pharmacol. 56, 199-228

Shimura, K., E. et al. (2008) J. Virol. 82, 764-774

Simon, V. et al. (2006) Lancet 368, 489-504

Sluis-Cremer, N., and Tachedjian, G. (2002) Eur. J. Biochem. 269, 5103-5111

Sluis-Cremer, N. et al. (2002) Mol.Pharmacol. 62, 398-405

Smolov, M. et al. (2006) FEBS J. 273, 1137-51

Sticht, J. et al. (2005) Nat. Struct. Mol. Biol. 12, 671-677

Tachedjian, G., and Goff, S. P. (2003) Curr. Opin. Investig. Drugs 4, 966-973

Tachedjian, G. et al. (2001) Proc. Natl. Acad. Sci. U S A 98, 7188-7193

Tachedjian, G. et al. (2005) FEBS Lett. 579, 379-384

Van Maele, B. and Debyser, Z. (2005) AIDS Rev. 7, 26-43.

Venezia, C. F. et al. (2006) Biochemistry 45, 2779-2789

Wain-Hobson, S. et al. (1985) Cell 40, 9-17

Wang, J. et al. (1994) Proc. Natl. Acad. Sci. U S A 91, 7242-7246

Wohrl, B. M. et al. (1997) J. Biol. Chem. 272, 17581-17587

Wohrl, B. M. et al. (1999) J. Mol. Biol. 292, 333-344

**Claims**

1. An isolated polypeptide, **characterized in that** it is selected amongst

   a) peptides consisting of or comprising the amino acid sequence $X_1X_2KWX_3TEX_4X_5PLX_6X_7X_8X_9X_{10}$ (SEQ ID NO: 17),
   wherein:

   $X_1$ is nothing or G,
   $X_2$ is nothing if $X_1$ is nothing, and $X_2$ is T if $X_1$ is G,
   $X_3$ is L or A
   $X_4$ is W or V,
   $X_5$ is I or A if $X_4$ is W, and $X_5$ is W if $X_4$ is V,
   $X_6$ is nothing or T,
   $X_7$ is nothing if $X_6$ is nothing, and $X_7$ is nothing or A if $X_6$ is T,
   $X_8$ is nothing if $X_7$ is nothing, and $X_8$ is E if $X_7$ is A,
   $X_9$ is A if $X_6$ is T, and $X_9$ is nothing if $X_6$ is nothing, and
   $X_{10}$ is E if $X_6$ is T, and $X_{10}$ is nothing if $X_6$ is nothing,
   and

   b) peptides consisting of the amino acid sequence SEQ ID NO: 1, or consisting of or comprising an amino acid sequence derived therefrom by the substitution of one of the amino acids at positions 1-3, 5, 6 and 8-14 of SEQ ID NO: 1 by an alanine (A) or a glycine (G), or the substitution of the amino acid at position 4 of SEQ ID NO: 1 by a glycine (G) or a valine (V),

   wherein said isolated polypeptide inhibits *in vitro* the HIV-1 Reverse Transcriptase polymerase more efficiently than the peptide Pep-A of amino acid sequence SEQ ID NO: 28.

2. A polypeptide according to claim 1, **characterized in that** it consists of or comprises the amino acid sequence $X_1X_2KWLTEX_3X_4PLX_5X_6X_7X_8X_9$ (SEQ ID NO: 34),
   wherein:

$X_1$ is nothing or G,
$X_2$ is nothing if $X_1$ is nothing, and $X_2$ is T if $X_1$ is G,
$X_3$ is W or V,
$X_4$ is I if $X_3$ is W, and $X_4$ is W if $X_3$ is V,
$X_5$ is nothing or T,
$X_6$ is nothing if $X_5$ is nothing, and $X_6$ is nothing or A if $X_5$ is T,
$X_7$ is nothing if $X_6$ is nothing, and $X_7$ is E if $X_6$ is A,
$X_8$ is A if $X_5$ is T, and $X_8$ is nothing if $X_5$ is nothing, and
$X_9$ is E if $X_5$ is T, and $X_9$ is nothing if $X_5$ is nothing.

3. A polypeptide according to claim 1, **characterized in that** the amino acid sequence corresponding to SEQ ID NO: 17 is the amino acid sequence SEQ ID NO: 26.

4. A polypeptide according to claim 1 or to claim 2, **characterized in that** the amino acid sequence corresponding to SEQ ID NO: 17 or SEQ ID NO: 34 is selected from the group consisting of the amino acid sequences SEQ ID NO: 18, 19, 23 to 25 and 27.

5. A polypeptide according to claim 1 or claim 2, **characterized in that** it further inhibits *in vitro* the HIV-1 integrase 3' processing activity.

6. A polypeptide according to claim 5, **characterized in that** it is selected from the group consisting of the amino acid sequences SEQ ID NO: 18, 19 and 24.

7. A polypeptide according to claim 1, **characterized in that** the amino acid sequence derived from SEQ ID NO: 1 is selected from the group consisting of SEQ ID NO: 2 to SEQ ID NO: 7, and SEQ ID NO: 9 to SEQ ID NO: 15.

8. A polypeptide according to any one of claims claim 1 to 7, **characterized in that** it further contains a cysteine residue at the N- or C-terminus.

9. A polypeptide consisting of the amino acid sequences SEQ ID NO 1 or an amino acid sequence derived therefrom, SEQ ID NO: 17 or SEQ ID NO: 34, as defined in any one of claims 1 to 7, **characterized in that**:

   - said polypeptide further contains a cysteine residue at the N-terminus or in the case where said polypeptide corresponds to SEQ ID NO: 17 or SEQ ID NO: 34 wherein $X_1$ and $X_2$ are respectively G and T, then the amino acid residue at position 2 of said polypeptide is substituted by a cysteine residue, and
   - said polypeptide further contains a cysteine residue at the C-terminus.

10. A polypeptide according to any one of claims claim 1 to 9, **characterized in that** one to three amino acid residues thereof is in D conformation.

11. A polypeptide according to any one of claims claim 1 to 10, **characterized in that** the N- or C-terminal amino acid residue thereof is a in beta conformation.

12. A polypeptide according to any one of claims 1 to 11, **characterized in that** it is coupled to a cell delivery agent.

13. A polypeptide according to claim 12, **characterized in that** the cell delivery agent is a peptide vector, preferably the peptides having the amino acid sequence SEQ ID NO: 29 (Pep-1) or SEQ ID NO: 33 (Pep-3).

14. A polypeptide according to any one of claims 1 to 13 for use as a medicament.

15. A polypeptide according to claim 14 as an antiviral agent.

16. A polypeptide according to claim 15 for treating or preventing a virus infection, preferably a HIV-1 virus infection.

17. A composition comprising at least one polypeptide as defined in any one of claims 1 to 13 and at least one pharmaceutically acceptable carrier.

18. Use of at least one polypeptide as defined in any one of claims 1 to 13 for inhibiting, *in vitro* or *ex vivo,* the reverse transcriptase polymerase activity of HIV-1 reverse transcriptase.

19. Use according to claim 18 for further inhibiting, *in vitro* or *ex vivo* the HIV-1 integrase 3' processing activity.

20. Use according to claim 19, **characterized** the polypeptide consists of or comprises the amino acid sequence selected from the group consisting of SEQ ID NO 18, 19 and 24.

21. An isolated polynucleotide encoding at least one polypeptide as defined in any one of claim 1 to 9.

22. A recombinant expression cassette, **characterized in that** it comprises a polynucleotide as defined in claim 21.

23. A recombinant vector, **characterized in that** it contains a recombinant expression cassette as defined in claim 22.

24. A host cell, **characterized in that** it contains a recombinant expression cassette of claim 20 or a recombinant vector of claim 23.

**Figure 1**

Figure 2

**A**

**B**

**Figure 3**

Figure 4

**Figure 5**

Figure 6

**Figure 7**

A

B

**Figure 8**

**Figure 9**

Figure 10

Europäisches
Patentamt

European
Patent Office

Office européen
des brevets

# EUROPEAN SEARCH REPORT

**Application Number**

EP 08 29 0908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | MORRIS M C ET AL: "THE THUMB DOMAIN OF THE P51-SUBUNIT IS ESSENTIAL FOR ACTIVATION OF HIV REVERSE TRANSCRIPTASE" BIOCHEMISTRY, AMERICAN CHEMICAL SOCIETY, EASTON, PA.; US, vol. 38, no. 46, 16 November 1999 (1999-11-16), pages 15097-15103, XP001153623 ISSN: 0006-2960 Abstract; Pep-A, which corresponds to present sequence 28, comprises present sequence 1. ----- | 1 | INV. C07K7/08 C07K14/16 A61P31/18 |
| X | EP 1 681 568 A (OKADA HIDECHIKA [JP]; OKADA NORIKO [JP]) 19 July 2006 (2006-07-19) Abstract; Table 1, peptide 283-302 comprises present sequence 1. ----- | 1 | |
| X | DATABASE UniProt [Online] 18 March 2008 (2008-03-18), "SubName: Full=Pol protein; Flags: Fragment;" XP002511117 retrieved from EBI accession no. UNIPROT:B0LXY1 Database accession no. B0LXY1 Comprises present sequence 3. * the whole document * ----- -/-- | 1 | TECHNICAL FIELDS SEARCHED (IPC) C07K |

The present search report has been drawn up for all claims

| Place of search | Date of completion of the search | Examiner |
|---|---|---|
| Munich | 20 January 2009 | López García, F |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

& : member of the same patent family, corresponding
 document

EPO FORM 1503 03.82 (P04C01)

**EUROPEAN SEARCH REPORT**

Application Number

EP 08 29 0908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online] 21 December 2004 (2004-12-21), "SubName: Full=Reverse transcriptase; Flags: Fragment;" XP002511118 retrieved from EBI accession no. UNIPROT:Q5RR69 Database accession no. Q5RR69 Comprises present sequence 5. * the whole document * ----- | 1 | |
| X | DATABASE UniProt [Online] 4 December 2007 (2007-12-04), "SubName: Full=Reverse transcriptase; Flags: Fragment;" XP002511119 retrieved from EBI accession no. UNIPROT:A8JWG4 Database accession no. A8JWG4 Comprises present sequence 11. * the whole document * ----- | 1 | |
| X | DATABASE UniProt [Online] 4 December 2007 (2007-12-04), "SubName: Full=Reverse transcriptase; Flags: Fragment;" XP002511120 retrieved from EBI accession no. UNIPROT:A8JWG7 Database accession no. A8JWG7 Comprises present sequence 14. * the whole document * ----- -/-- | 1 | **TECHNICAL FIELDS SEARCHED (IPC)** |

The present search report has been drawn up for all claims

| Place of search | Date of completion of the search | Examiner |
|---|---|---|
| Munich | 20 January 2009 | López García, F |

EPO FORM 1503 03.82 (P04C01)

# EP 2 174 948 A1

Europäisches
Patentamt

European
Patent Office

Office européen
des brevets

## EUROPEAN SEARCH REPORT

Application Number

EP 08 29 0908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online] 10 June 2008 (2008-06-10), "SubName: Full=Pol protein; Flags: Fragment;" XP002511121 retrieved from EBI accession no. UNIPROT:B2M3Q9 Database accession no. B2M3Q9 Comprises present sequence 15. * the whole document * ----- | 1 | |
| A | WO 02/15661 A (CENTRE NAT RECH SCIENT [FR]; DEVAUX CHRISTIAN [FR]; HEBMANN VERONIQUE) 28 February 2002 (2002-02-28) Abstract; Tble 1, p. 16. ----- | 1-24 | |
| A | AGOPIAN ET AL: "p66 Trp24 and Phe61 Are Essential for Accurate Association of HIV-1 Reverse Transcriptase with Primer/Template" JOURNAL OF MOLECULAR BIOLOGY, LONDON, GB, vol. 373, no. 1, 14 September 2007 (2007-09-14), pages 127-140, XP022250535 ISSN: 0022-2836 * abstract * ----- | 1-24 | |
| T | AGOPIAN AUDREY ET AL: "A New Generation of Peptide-based Inhibitors Targeting HIV-1 Reverse Transcriptase Conformational Flexibility." THE JOURNAL OF BIOLOGICAL CHEMISTRY 2 JAN 2009, vol. 284, no. 1, 2 January 2009 (2009-01-02), pages 254-264, XP002511116 ISSN: 0021-9258 * the whole document * ----- | | TECHNICAL FIELDS SEARCHED (IPC) |

The present search report has been drawn up for all claims

| Place of search | Date of completion of the search | Examiner |
|---|---|---|
| Munich | 20 January 2009 | López García, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

49

## ANNEX TO THE EUROPEAN SEARCH REPORT
## ON EUROPEAN PATENT APPLICATION NO.

EP 08 29 0908

This annex lists the patent family members relating to the patent documents cited in the above-mentioned European search report.
The members are as contained in the European Patent Office EDP file on
The European Patent Office is in no way liable for these particulars which are merely given for the purpose of information.

20-01-2009

| Patent document cited in search report | | Publication date | Patent family member(s) | | Publication date |
|---|---|---|---|---|---|
| EP 1681568 | A | 19-07-2006 | CN<br>WO<br>US | 1871515 A<br>2005040799 A1<br>2006275826 A1 | 29-11-2006<br>06-05-2005<br>07-12-2006 |
| WO 0215661 | A | 28-02-2002 | AU<br>EP | 9215301 A<br>1311538 A2 | 04-03-2002<br>21-05-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Agopian, A. et al.** *J. Mol. Biol.,* 2007, vol. 373, 127-140
- **Anker, M. ; Corales, R. B.** *Expert Opin. Investig. Drugs,* 2008, vol. 17, 97-103
- **Barre-Sinoussi, F. et al.** *Science,* 1983, vol. 220, 868-871
- **Bukrinsky, M. I. et al.** *Proc. Natl. Acad. Sci. U S A,* 1992, vol. 89, 6580-6584
- **Cabodevilla, J. F. et al.** *Eur. J. Biochem.,* 2001, vol. 268, 1163-1172
- **Camarasa, M. J. et al.** *Antiviral Res.,* 2006, vol. 71, 260-267
- **Cherepanov, P. et al.** *J Biol. Chem.,* 2003, vol. 278, 372-81
- **Craigie, R.** *J Biol. Chem.,* 2001, vol. 276, 23213-6
- **De Clercq, E.** *Nat. Rev. Drug Discov.,* 2007, vol. 6, 1001-1018
- **De Coupade, C. et al.** *Biochem. J.,* 2005, vol. 390, 407-18
- **Depienne, C. et al.** *Experimental Cell Research,* 2000, vol. 260, 387-395
- **Depienne, C. et al.** *J. Biol. Chem.,* 2001, vol. 276, 18102-18107
- **Depollier, J. et al.** *Biochemistry,* 2005, vol. 44, 1909-1918
- **Deprez, E. et al.** *Proc. Natl. Acad. Sci. U S A,* 2001, vol. 98, 10090-10095
- **Deprez, E. et al.** *Mol. Pharmacol.,* 2004, vol. 65, 85-98
- **Derossi, D. et al.** *J Biol. Chem.,* 1996, vol. 271, 18188-18193
- **Di Marzo Veronese, F. et al.** *Science,* 1986, vol. 231, 1289-1291
- **Divita, G. et al.** *J. Biol. Chem.,* 1994, vol. 269, 13080-13083
- **Divita, G. et al.** *J. Biol. Chem.,* 1995, vol. 270, 28642-28646
- **Divita, G. et al.** *J. Mol. Biol.,* 1995, vol. 245, 508-521
- **Divita, G. et al.** *Biochemistry,* 1995, vol. 34, 16337-16346
- **Dyda, F. et al.** *Science,* 1994, vol. 266, 1981-6
- **Elliott, G. ; O'Hare, P.** *Cell,* 1997, vol. 88, 223-233
- **Farnet, C. M. et al.** *Cell,* 1997, vol. 88, 483-92
- **Figueiredo, A. et al.** *PLoS pathogens,* 2006, vol. 2, e 119
- **Fisher, T. S. ; Prasad, V. R.** *J. Biol. Chem.,* 2002, vol. 277, 22345-22352
- **Fisher, T. S. ; Darden, T. ; Prasad, V. R.** *J. Mol. Biol.,* 2003, vol. 325, 443-459
- **Ghosh, M. et al.** *Biochemistry,* 1996, vol. 35, 8553-8562
- **Grinsztejn, B. et al.** *Lancet,* 2007, vol. 369, 1261-9
- **Gros, E. et al.** *Biochimic et biophysica acta,* 2006, vol. 1758, 384-393
- **Guiot, E. et al.** *J Biol. Chem.,* 2006, vol. 281, 22707-22719
- **Hsiou, Y. et al.** *Structure,* 1996, vol. 4, 853-860
- **Huang, H. et al.** *Science,* 1998, vol. 282, 1669-1675
- **Huang, S. C. et al.** *Biochem. Biophys. Res. Commun.,* 1992, vol. 184, 986-992
- **Jacobo-Molina, A. et al.** *Proc. Natl. Acad. Sci. USA,* 1993, vol. 90, 6320-6324
- **Kärber.** *Naunyn-Schmiedebergs Archiv für experimentelle Pathologie und Pharmakologie,* 1931, vol. 162, 480-483
- **Kohlstaedt, L. A. et al.** *Science,* 1992, vol. 256, 1783-1790
- **Kokryakov, V .N. et al.** *FEBS Lett.,* 1993, vol. 327, 231-236
- **Lightfoote, M. M. et al.** *J. Virol.,* 1986, vol. 60, 771-775
- **Malet, I. et al.** *Agents Chemother.,* 2008, vol. 52, 1351-1358
- **Matsuzaki, Y. et al.** *XIIIth CROI,* 05 February 2006
- **Morris, M. C. et al.** *Biochemistry,* 1999, vol. 38, 15097-15103
- **Morris, M. C. et al.** *J. Biol. Chem.,* 1999, vol. 274, 24941-24946
- **Morris, M. C. et al.** *Nat.Biotech.,* 2001, vol. 19, 1173-1176
- **Morris, M. C. et al.** *Nucleic Acids Res.,* 2007, vol. 35, e49
- **Mossmann T.** *J. Immunol. Methods,* 1983, vol. 65, 55-63
- **Mousnier, A. et al.** *Proc. Natl. Acad. Sci. U S A,* 2007, vol. 104, 13615-20
- **Muller, B. et al.** *J. Biol. Chem.,* 1989, vol. 264, 13975-13978
- **Patel, P. H. et al.** *Biochemistry,* 1995, vol. 34, 5351-5363

- **Piller, S. C. et al.** *Curr. Drug Targets,* 2003, vol. 4, 409-29
- **Pommier, Y. et al.** *Nat. Rev. Drug Discov.,* 2005, vol. 4, 236-48
- **Popov, S. et al.** *EMBO J.,* 1998, vol. 17, 909-17
- **Restle, T. et al.** *J. Biol. Chem.,* 1990, vol. 265, 8986-8988
- **Rittinger, K. et al.** *Proc. Natl. Acad. Sci. U S A,* 1995, vol. 92, 8046-8049
- **Rodgers, D. W. et al.** *Proc. Natl. Acad. Sci. USA,* 1995, vol. 92, 1222-1226
- **Roisin, A. et al.** *J. Biol. Chem.,* 2004, vol. 279, 9208-9214
- **Ruben, S. et al.** *J. Virol.,* 1989, vol. 63, 1-8
- **Sato, M. et al.** *J. Med. Chem.,* 2006, vol. 49, 1506-1508
- **Semenova, E. A. et al.** *Adv. Pharmacol.,* 2008, vol. 56, 199-228
- **Shimura, K., E. et al.** *J. Virol.,* 2008, vol. 82, 764-774
- **Simon, V. et al.** *Lancet,* 2006, vol. 368, 489-504
- **Sluis-Cremer, N. ; Tachedjian, G.** *Eur. J. Biochem.,* 2002, vol. 269, 5103-5111
- **Sluis-Cremer, N. et al.** *Mol.Pharmacol.,* 2002, vol. 62, 398-405
- **Smolov, M. et al.** *FEBS J.,* 2006, vol. 273, 1137-51
- **Sticht, J. et al.** *Nat. Struct. Mol. Biol.,* 2005, vol. 12, 671-677
- **Tachedjian, G. ; Goff, S. P.** *Curr. Opin. Investig. Drugs,* 2003, vol. 4, 966-973
- **Tachedjian, G. et al.** *Proc. Natl. Acad. Sci. U S A,* 2001, vol. 98, 7188-7193
- **Tachedjian, G. et al.** *FEBS Lett.,* 2005, vol. 579, 379-384
- **Van Maele, B. ; Debyser, Z.** *AIDS Rev.,* 2005, vol. 7, 26-43
- **Venezia, C. F. et al.** *Biochemistry,* 2006, vol. 45, 2779-2789
- **Wain-Hobson, S. et al.** *Cell,* 1985, vol. 40, 9-17
- **Wang, J. et al.** *Proc. Natl. Acad. Sci. U S A,* 1994, vol. 91, 7242-7246
- **Wohrl, B. M. et al.** *J. Biol. Chem.,* 1997, vol. 272, 17581-17587
- **Wohrl, B. M. et al.** *J. Mol. Biol.,* 1999, vol. 292, 333-344

SEQUENCE LISTING

<110> CENTRE NATIONAL DE LA RECHERCHE SCIENTIFIQUE
DIVITA, Gilles
AGOPIAN, Audrey
GROS, Edwige

<120> ANTIVIRAL POLYPEPTIDES

<130> XRN/ahF644-185-186EP

<160> 40

<170> PatentIn version 3.3

<210> 1
<211> 16
<212> PRT
<213> Artificial

<220>
<223> peptide derived from HIV-1 reverse transcriptase

<400> 1

Gly Thr Lys Ala Leu Thr Glu Val Ile Pro Leu Thr Glu Glu Ala Glu
1               5                   10                  15


<210> 2
<211> 16
<212> PRT
<213> Artificial

<220>
<223> peptide derived from HIV-1 reverse transcriptase

<400> 2

Ala Thr Lys Ala Leu Thr Glu Val Ile Pro Leu Thr Glu Glu Ala Glu
1               5                   10                  15


<210> 3
<211> 16
<212> PRT
<213> Artificial

<220>
<223> peptide derived from HIV-1 reverse transcriptase

<400> 3

Gly Ala Lys Ala Leu Thr Glu Val Ile Pro Leu Thr Glu Glu Ala Glu
1               5                   10                  15


<210> 4
<211> 16
<212> PRT
<213> Artificial

<220>
<223> peptide derived from HIV-1 reverse transcriptase

<400> 4

Gly Thr Ala Ala Leu Thr Glu Val Ile Pro Leu Thr Glu Glu Ala Glu
1               5               10              15

<210>   5
<211>   16
<212>   PRT
<213>   Artificial

<220>
<223>   peptide derived from HIV-1 reverse transcriptase

<400>   5

Gly Thr Lys Gly Leu Thr Glu Val Ile Pro Leu Thr Glu Glu Ala Glu
1               5               10              15

<210>   6
<211>   16
<212>   PRT
<213>   Artificial

<220>
<223>   peptide derived from HIV-1 reverse transcriptase

<400>   6

Gly Thr Lys Ala Ala Thr Glu Val Ile Pro Leu Thr Glu Glu Ala Glu
1               5               10              15

<210>   7
<211>   16
<212>   PRT
<213>   Artificial

<220>
<223>   peptide derived from HIV-1 reverse transcriptase

<400>   7

Gly Thr Lys Ala Leu Ala Glu Val Ile Pro Leu Thr Glu Glu Ala Glu
1               5               10              15

<210>   8
<211>   16
<212>   PRT
<213>   Artificial

<220>
<223>   peptide derived from HIV-1 reverse transcriptase

<400>   8

Gly Thr Lys Ala Leu Thr Ala Val Ile Pro Leu Thr Glu Glu Ala Glu
1               5               10              15

<210>   9
<211>   16
<212>   PRT
<213>   Artificial

```
<220>
<223>   peptide derived from HIV-1 reverse transcriptase

<400>   9

Gly Thr Lys Ala Leu Thr Glu Ala Ile Pro Leu Thr Glu Glu Ala Glu
1               5                   10                  15


<210>   10
<211>   16
<212>   PRT
<213>   Artificial

<220>
<223>   peptide derived from HIV-1 reverse transcriptase

<400>   10

Gly Thr Lys Ala Leu Thr Glu Val Ala Pro Leu Thr Glu Glu Ala Glu
1               5                   10                  15


<210>   11
<211>   16
<212>   PRT
<213>   Artificial

<220>
<223>   peptide derived from HIV-1 reverse transcriptase

<400>   11

Gly Thr Lys Ala Leu Thr Glu Val Ile Ala Leu Thr Glu Glu Ala Glu
1               5                   10                  15


<210>   12
<211>   16
<212>   PRT
<213>   Artificial

<220>
<223>   peptide derived from HIV-1 reverse transcriptase

<400>   12

Gly Thr Lys Ala Leu Thr Glu Val Ile Pro Ala Thr Glu Glu Ala Glu
1               5                   10                  15


<210>   13
<211>   16
<212>   PRT
<213>   Artificial

<220>
<223>   peptide derived from HIV-1 reverse transcriptase

<400>   13

Gly Thr Lys Ala Leu Thr Glu Val Ile Pro Leu Ala Glu Glu Ala Glu
1               5                   10                  15


<210>   14
```

```
<211>   16
<212>   PRT
<213>   Artificial

<220>
<223>   peptide derived from HIV-1 reverse transcriptase

<400>   14

Gly Thr Lys Ala Leu Thr Glu Val Ile Pro Leu Thr Ala Glu Ala Glu
1               5               10              15


<210>   15
<211>   16
<212>   PRT
<213>   Artificial

<220>
<223>   peptide derived from HIV-1 reverse transcriptase

<400>   15

Gly Thr Lys Ala Leu Thr Glu Val Ile Pro Leu Thr Glu Ala Ala Glu
1               5               10              15


<210>   16
<211>   16
<212>   PRT
<213>   Artificial

<220>
<223>   Srambled peptide

<400>   16

Gly Ala Lys Thr Glu Thr Leu Val Ile Pro Glu Thr Glu Leu Glu Ala
1               5               10              15


<210>   17
<211>   16
<212>   PRT
<213>   Artificial

<220>
<223>   peptide derived from HIV-1 reverse transcriptase


<220>
<221>   VARIANT
<222>   (1)..(1)
<223>   /replace by nothing

<220>
<221>   VARIANT
<222>   (2)..(2)
<223>   /replace by nothing if amino acid 1 is nothing

<220>
<221>   VARIANT
<222>   (5)..(5)
<223>   /replace by Ala

<220>
```

```
<221>   VARIANT
<222>   (8)..(8)
<223>   /replace by Val

<220>
<221>   VARIANT
<222>   (9)..(9)
<223>   /replace by Ala if amino acid 8 is Trp, and by Trp if amino acid
        8 is Val

<220>
<221>   VARIANT
<222>   (12)..(12)
<223>   /replace by nothing

<220>
<221>   VARIANT
<222>   (13)..(13)
<223>   /replace by nothing if amino acid 12 is nothing

<220>
<221>   VARIANT
<222>   (14)..(14)
<223>   /replace by nothing if amino acid 13 is nothing

<220>
<221>   VARIANT
<222>   (15)..(15)
<223>   /replace by nothing

<220>
<221>   VARIANT
<222>   (16)..(16)
<223>   /replace by nothing

<400>   17

Gly Thr Lys Trp Leu Thr Glu Trp Ile Pro Leu Thr Ala Glu Ala Glu
1                   5                   10                  15


<210>   18
<211>   16
<212>   PRT
<213>   Artificial

<220>
<223>   peptide derived from HIV-1 reverse transcriptase

<400>   18

Gly Thr Lys Trp Leu Thr Glu Trp Ile Pro Leu Thr Ala Glu Ala Glu
1                   5                   10                  15


<210>   19
<211>   11
<212>   PRT
<213>   Artificial

<220>
<223>   peptide derived from HIV-1 reverse transcriptase

<400>   19

Gly Thr Lys Trp Leu Thr Glu Val Trp Pro Leu
```

<210> 20
<211> 11
<212> PRT
<213> Artificial

<220>
<223> peptide derived from HIV-1 reverse transcriptase

<400> 20

Gly Thr Lys Ala Trp Thr Glu Val Trp Pro Leu

<210> 21
<211> 12
<212> PRT
<213> Artificial

<220>
<223> peptide derived from HIV-1 reverse transcriptase

<400> 21

Gly Thr Lys Ala Leu Thr Glu Val Ile Pro Leu Thr

<210> 22
<211> 12
<212> PRT
<213> Artificial

<220>
<223> peptide derived from HIV-1 reverse transcriptase

<400> 22

Gly Thr Lys Ala Ala Thr Glu Val Ile Pro Leu Thr

<210> 23
<211> 11
<212> PRT
<213> Artificial

<220>
<223> peptide derived from HIV-1 reverse transcriptase

<400> 23

Gly Thr Lys Trp Leu Thr Glu Trp Ile Pro Leu

<210> 24
<211> 14
<212> PRT
<213> Artificial

<220>
<223> peptide derived from HIV-1 reverse transcriptase

<400> 24

Lys Trp Leu Thr Glu Trp Ile Pro Leu Thr Ala Glu Ala Glu
1               5               10

<210> 25
<211> 14
<212> PRT
<213> Artificial

<220>
<223> peptide derived from HIV-1 reverse transcriptase

<400> 25

Gly Thr Lys Trp Leu Thr Glu Trp Ile Pro Leu Thr Ala Glu
1               5               10

<210> 26
<211> 16
<212> PRT
<213> Artificial

<220>
<223> peptide derived from HIV-1 reverse transcriptase

<400> 26

Gly Thr Lys Trp Ala Thr Glu Trp Ala Pro Leu Thr Ala Glu Ala Glu
1               5               10                  15

<210> 27
<211> 12
<212> PRT
<213> Artificial

<220>
<223> peptide derived from HIV-1 reverse transcriptase

<400> 27

Lys Trp Leu Thr Glu Trp Ile Pro Leu Thr Ala Glu
1               5               10

<210> 28
<211> 17
<212> PRT
<213> Artificial

<220>
<223> peptide derived from HIV-1 reverse transcriptase

<400> 28

Arg Gly Thr Lys Ala Leu Thr Glu Val Ile Pro Leu Thr Glu Glu Ala
1               5               10                  15

Glu

```
<210>  29
<211>  21
<212>  PRT
<213>  Artificial

<220>
<223>  peptide vector

<400>  29

Lys Glu Thr Trp Trp Glu Thr Trp Trp Thr Glu Trp Ser Gln Pro Lys
1               5                   10                  15


Lys Lys Arg Lys Val
            20


<210>  30
<211>  19
<212>  DNA
<213>  Artificial

<220>
<223>  primer strand

<400>  30
tccctgttcg ggcgccact                                                    19


<210>  31
<211>  36
<212>  DNA
<213>  Artificial

<220>
<223>  template strand

<400>  31
tgtggaaaat ctcatgcagt ggcgcccgaa caggga                                36


<210>  32
<211>  10
<212>  PRT
<213>  Artificial

<220>
<223>  Pep-7 ; described in Morris et al., 1999, JBC, 274, 24941-24946

<400>  32

Lys Glu Thr Trp Glu Thr Trp Trp Thr Glu
1               5                   10


<210>  33
<211>  15
<212>  PRT
<213>  Artificial

<220>
<223>  peptide vector

<400>  33
```

```
Lys Trp Phe Glu Thr Trp Phe Thr Glu Trp Pro Lys Lys Arg Lys
1               5                   10                  15
```

```
<210>   34
<211>   16
<212>   PRT
<213>   Artificial

<220>
<223>   peptide derived from HIV-1 reverse transcriptase


<220>
<221>   VARIANT
<222>   (1)..(1)
<223>   /replace by nothing

<220>
<221>   VARIANT
<222>   (2)..(2)
<223>   /replace by nothing if amino acid 1 is nothing

<220>
<221>   VARIANT
<222>   (8)..(8)
<223>   /replace by Val

<220>
<221>   VARIANT
<222>   (9)..(9)
<223>   /replace by Trp if amino acid 8 is Val

<220>
<221>   VARIANT
<222>   (12)..(12)
<223>   /replace by nothing

<220>
<221>   VARIANT
<222>   (13)..(13)
<223>   /replace by nothing if amino acid 12 is nothing

<220>
<221>   VARIANT
<222>   (14)..(14)
<223>   /replace by nothing if amino acid 13 is nothing

<220>
<221>   VARIANT
<222>   (15)..(15)
<223>   /replace by nothing

<220>
<221>   VARIANT
<222>   (16)..(16)
<223>   /replace by nothing

<400>   34
```

```
Gly Thr Lys Trp Leu Thr Glu Trp Ile Pro Leu Thr Ala Glu Ala Glu
1               5                   10                  15
```

```
<210>   35
```

```
<211>    18
<212>    PRT
<213>    Artificial

<220>
<223>    Cyclized Paw peptide

<400>    35
```

Cys Gly Thr Lys Trp Leu Thr Glu Trp Ile Pro Leu Thr Ala Glu Ala

Glu Cys

```
<210>    36
<211>    17
<212>    PRT
<213>    Artificial

<220>
<223>    Cyclized Paw peptide

<400>    36
```

Gly Cys Lys Trp Leu Thr Glu Trp Ile Pro Leu Thr Ala Glu Ala Glu

Cys

```
<210>    37
<211>    13
<212>    PRT
<213>    Artificial

<220>
<223>    Cyclized P16 peptide

<400>    37
```

Cys Gly Thr Lys Trp Leu Thr Glu Val Trp Pro Leu Cys

```
<210>    38
<211>    12
<212>    PRT
<213>    Artificial

<220>
<223>    Cyclized P16 peptide

<400>    38
```

Gly Cys Lys Trp Leu Thr Glu Val Trp Pro Leu Cys

```
<210>    39
<211>    16
<212>    PRT
```

```
<213>   Artificial

<220>
<223>   Cyclized P27 peptide

<400>   39

Cys Gly Thr Lys Trp Leu Thr Glu Trp Ile Pro Leu Thr Ala Glu Cys
1               5                   10                  15


<210>   40
<211>   15
<212>   PRT
<213>   Artificial

<220>
<223>   Cyclized P27 peptide

<400>   40

Gly Cys Lys Trp Leu Thr Glu Trp Ile Pro Leu Thr Ala Glu Cys
1               5                   10                  15
```